# EUROPEAN PATENT SPECIFICATION

(11) **EP 4 577 293 B1**
(45) Date of publication and mention of the grant of the patent: **24.06.2026**
(21) Application number: 23777452.6
(22) Date of filing: 24.08.2023
(51) Int. Cl.: A61N 1/05, A61B 5/293, A61B 5/00

(54) **ENDOVASCULAR DEEP BRAIN STIMULATION**
ENDOVASKULÄRE TIEFENSTIMULATION DES GEHIRNS
STIMULATION CÉRÉBRALE PROFONDE ENDOVASCULAIRE

(30) Priority: 26.08.2022 US 202263373606 P; 26.08.2022 US 202263373612 P; 09.02.2023 US 202363484049 P; 23.08.2023 US 202318454428
(43) Date of publication of application: 02.07.2025
(73) Proprietor: Covidien LP, Mansfield, MA 02048 (US)
(72) Inventor: NGUYEN, Kevin V., Irvine, California 92617 (US); ASHBY, Mark P., Irvine, California 92617 (US); KASHYAP, Varun Umesh, Irvine, California 92617 (US); HUYNH, Andyanhdzung, Irvine, California 92617 (US); STANSLASKI, Scott R., Minneapolis, Minnesota 55432 (US)
(74) Representative: Gray, James
(86) International application number: PCT/US2023/072808
(87) International publication number: WO 2024/044678

(56) References cited:
- US-A1- 2005 137 647
- US-A1- 2018 236 221
- US-A1- 2022 240 833

## Description

### TECHNICAL FIELD

This disclosure relates to electrical stimulation therapy.

### BACKGROUND

Medical devices, such as electrical stimulation devices, may be used in different therapeutic applications, such as deep brain stimulation (DBS). A medical device may be used to deliver therapy to a patient to treat a variety of symptoms or patient conditions such as movement disorders, seizure disorders (e.g., epilepsy), or mood disorders. In some therapy systems, an implantable electrical stimulator delivers electrical stimulation therapy to a target tissue site within a patient with the aid of one or more electrodes.
US2018/236221 describes devices, methods and systems for transmitting signals through a device located in a blood vessel of an animal, for stimulating and/or sensing activity of media proximal to the device, wherein the media includes tissue and/or fluid.

### SUMMARY

The invention is defined by independent claim 1. This disclosure describes example endovascular medical devices and systems configured to deliver electrical stimulation therapy to a brain of a patient and/or sense one or more patient parameters (e.g., brain signals and/or other physiological parameters), as well as non-claimed methods of delivering endovascular deep brain stimulation (DBS) or sensing using the devices and systems described herein. The medical devices and systems described herein are configured to be relatively minimally invasive at least because they are configured to be navigated through vasculature of a patient to a cranial blood vessel (e.g., a venous vessel or an arterial vessel) in order to deliver electrical stimulation therapy to a target tissue site in a brain of a patient.

In the examples described herein, an endovascular device includes an elongated body (e.g., a lead body or a catheter body), and one or more expandable elements (e.g., including one or more loops, hoops, rings, or other shapes) including one or more electrodes. The one or more expandable elements are configured to expand radially outwards from a relatively low-profile delivery configuration to a deployed configuration to position one or more electrodes to deliver electrical stimulation to tissue of a brain of a patient or sense a patient parameter from a location within a cranial blood vessel of the patient. The configurations of the endovascular devices including one or more expandable elements described herein have low profiles which may facilitate delivery, placement, and/or deployment of electrodes in the vasculature of the brain while minimizing adverse effects, including thrombosis. For example, the endovascular devices including one or more expandable elements described herein may have lower metal coverage and/or cross-sectional area as compared to other means of deploying electrodes within cranial blood vessels (e.g., stents). Additionally, the endovascular devices described herein may be configured for directional stimulation and/or directional sensing (e.g., electrodes that face in particular directions or are otherwise configured to facilitate directional stimulation and/or directional sensing).

In some examples, an endovascular medical device includes an elongated body configured to be introduced in a cranial blood vessel of a patient and an expandable element on the elongated body. The expandable element includes a plurality of loops, hoops, rings, or other shapes. The endovascular device includes one or more electrodes (e.g., on the expandable element) configured to deliver electrical stimulation therapy or sense a patient parameter. The expandable element is configured to expand radially outwards from a relatively low-profile delivery configuration to a deployed configuration to position the one or more electrodes to deliver electrical stimulation to tissue of a brain of a patient or sense a patient parameter from a location within the cranial blood vessel.

In some examples, an endovascular medical device includes an elongated body including an expandable element with one or more electrodes on the expandable element. The expandable element includes a plurality of loops. Each loop of the plurality of loops defines a flower petal shape. Each loop of the plurality of loops extends radially outward from a common location from the elongated body. The expandable element is configured to expand radially outwards from a relatively low-profile delivery configuration to a deployed configuration to position the one or more electrodes to deliver electrical stimulation to tissue of a brain of a patient or sense a patient parameter from a location within a cranial blood vessel of the patient.

In some examples, an endovascular medical device includes an elongated body comprising a plurality of expandable elements and one or more electrodes on each expandable element of the plurality of expandable elements. Each expandable element of the plurality of expandable elements includes a loop. Each expandable element of the plurality of expandable elements are longitudinally separated from each other along a longitudinal axis of the endovascular medical device. The expandable element is configured to expand radially outwards from a relatively low-profile delivery configuration to a deployed configuration to position the one or more electrodes to deliver electrical stimulation to tissue of a brain of a patient or sense a patient parameter from a location within a cranial blood vessel of the patient.

The examples described herein may be combined in any permutation or combination.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 is a conceptual diagram illustrating an example therapy system including an endovascular device configured to deliver electrical stimulation therapy to a target tissue site in a brain of a patient.
FIG. 2 shows example vasculature of a brain of a patient.
FIG. 3 is a functional block diagram illustrating components of an example medical device of the therapy system of FIG. 1.
FIG. 4A illustrates an example endovascular device of the therapy system of FIG. 1.
FIG. 4B illustrates an example endovascular device of the therapy system of FIG. 1.
FIG. 4C illustrates an end view of an example endovascular device of the therapy system of FIG. 1.
FIG. 4D illustrates an end view of an example endovascular device of the therapy system of FIG. 1.
FIG. 5A illustrates an example endovascular device of the therapy system of FIG. 1.
FIG. 5B illustrates another view of the example endovascular device of FIG. 5A.
FIG. 6A illustrates an example endovascular device of the therapy system of FIG. 1.
FIG. 6B illustrates an end view of the example endovascular device of FIG. 6A.
FIG. 7 illustrates an example technique for defining electrodes of an endovascular device by removing electrically insulative material to expose an electrically conductive material.
FIG. 8A illustrates example electrodes with insulative material covering a portion of the electrodes.
FIG. 8B illustrates a cross section of an example electrode from FIG. 8A.
FIG. 9 is a conceptual diagram illustrating an example electrical stimulation waveform.
FIG. 10 is a conceptual diagram illustrating another example electrical stimulation waveform.
FIG. 11 is a conceptual diagram illustrating further example electrical stimulation waveforms.
FIG. 12 is a flow diagram of a technique for delivering endovascular deep brain stimulation.

### DETAILED DESCRIPTION

The disclosure describes devices, systems, and methods relating to delivery of electrical stimulation therapy, such as deep brain stimulation (DBS), or sensing one or more patient parameters (e.g., brain signals and/or other physiological parameters, such as cardiac signals, impedance, electroencephalogram (EEG), evoked potentials, local field potentials, etc.) from an endovascular location. Example endovascular locations that can be used to access the brain sites for electrical stimulation therapy or sensing using the devices described herein include any suitable cranial blood vessel (also referred to herein as a cerebral blood vessel or neurovasculature, which can include a vein or an cranial artery), such as, but not limited to, the thalamostriate vein, the internal cerebral vein, the basal vein of Rosenthal, the inferior sagittal sinus, the superior sagittal sinus, or the anterior choroidal artery.

DBS has been proposed for use to manage one or more patient conditions, such as to treat the patient condition by reducing or even eliminating one or more symptoms associated with the patient condition. For example, DBS can be used to alleviate, and in some cases, eliminate symptoms associated with movement disorders, other neurodegenerative impairment, seizure disorders, psychiatric disorders (e.g., mood disorders), or the like. Movement disorders may be found in patients with Parkinson's disease, multiple sclerosis, and cerebral palsy, among other conditions, and can be associated with disease or trauma. DBS can be delivered to one or more target sites in a brain of a patient to help a patient with muscle control and minimize movement problems, such as rigidity, bradykinesia (i.e., slow physical movement), rhythmic hyperkinesia (e.g., tremor), nonrhythmic hyperkinesia (e.g., tics) or akinesia (i.e., a loss of physical movement).

In the case of seizure disorders, DBS can be delivered to one or more target sites in a brain of a patient to reduce the frequency or severity of seizures, or even help prevent the occurrence of seizures. In the case of psychiatric disorders, DBS can be delivered to help minimize or even eliminate symptoms associated with major depressive disorder (MDD), bipolar disorder, anxiety disorders, post-traumatic stress disorder, dysthymic disorder, or obsessive-compulsive disorder (OCD). DBS can also reduce the symptoms of Parkinson's disease, dystonia, or cerebellar outflow tremor.

In the examples described herein, an endovascular device includes an elongated body (e.g., a lead body or a catheter body), and one or more expandable elements (e.g., including one or more loops, hoops, rings, or other shapes) including one or more electrodes. The one or more expandable elements are configured to expand radially outwards from a relatively low-profile delivery configuration to a deployed configuration to position one or more electrodes to deliver electrical stimulation to tissue of a brain of a patient or sense a patient parameter from a location within a cranial blood vessel of the patient. The configurations of the endovascular devices including one or more expandable elements described herein (e.g., one or more loops) have low profiles which may facilitate delivery, placement, and/or deployment of electrodes in the vasculature of the brain while minimizing adverse effects, including thrombosis. For example, the endovascular devices including one or more expandable elements described herein (e.g., one or more loops) may have lower metal coverage and/or cross-sectional area as compared to other means of deploying electrodes within cranial blood vessels (e.g., stents).

In some examples, the elongated body is configured to be directly or indirectly electrically connected to a medical device that is configured to generate the electrical stimulation and/or sense a patient parameter via the one or more electrodes of the endovascular device. In other examples, the elongated body is a wireless therapy delivery device, such as a microstimulator or the like, which does not need to be electrically coupled to a separate medical device to deliver electrical stimulation therapy and/or sense a patient parameter.

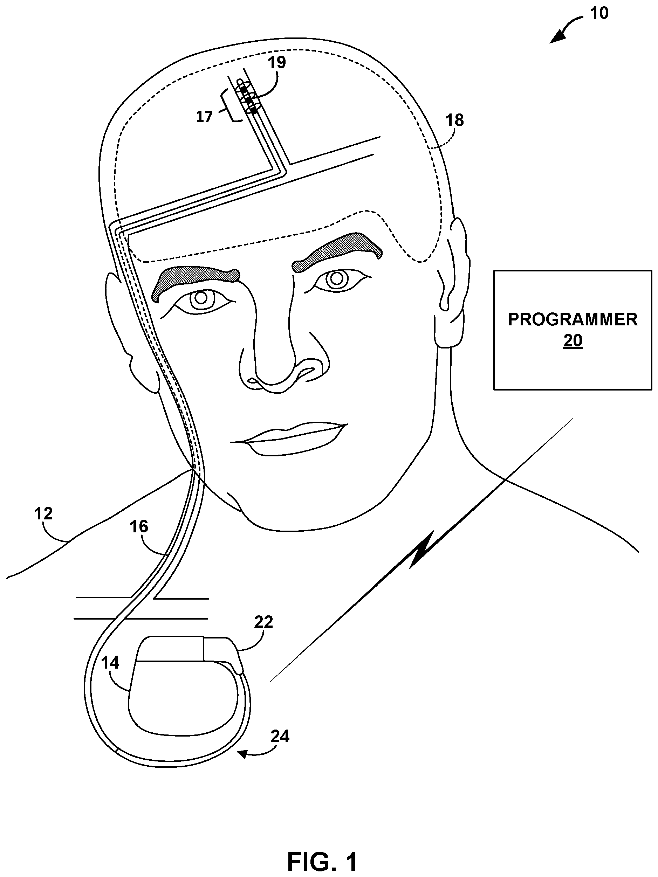

FIG. 1 is a conceptual diagram illustrating an example therapy system 10 configured to deliver electrical stimulation therapy to a target tissue site in a brain of a patient 12. Patient 12 ordinarily will be a human patient. In some cases, however, therapy system 10 can be applied to other mammalian or non-mammalian non-human patients. Therapy system 10 includes a medical device 14 and an endovascular therapy delivery device 16 (also referred to herein as an "endovascular device 16" or a "therapy delivery device 16"). In the example shown in FIG. 1, medical device 14 is configured to deliver electrical stimulation therapy (e.g., DBS) to a brain 18 of patient 12 and/or sense bioelectrical brain signals in brain 18 via electrodes 17. Endovascular device 16 is positioned in cranial vasculature of patient 12 such that one or more electrodes 17 are located proximate to a target tissue site within brain 18 and are positioned to deliver electrical stimulation therapy to and/or sense signals from brain sites within brain 18, such as tissue sites under the dura mater surrounding brain 18. These tissue sites may also be referred to as deep brain tissue sites. Endovascular device 16 includes an expandable element 19 which may help hold electrodes 17 in apposition with a vessel wall. Medical device 14 can provide electrical stimulation to one or more regions within brain 18 in order to manage a condition of patient 12, such as to mitigate the severity or duration of the patient condition.

Endovascular device 16 includes any suitable medical device configured to deliver electrical stimulation signals to tissue proximate electrodes 17. For example, endovascular device 16 can be a medical lead, a catheter, a guidewire, or another elongated body carrying electrodes 17 and configured to be electrically coupled to medical device 14 either directly or indirectly via an electrically conductive pathway that runs between medical device 14 and electrodes 17. Endovascular device 16 has any suitable length that enables connection to medical device 14 either directly or indirectly, e.g., a length of 150 centimeters (cm) to 250 cm, such as 200 cm. Further, endovascular device 16 has a suitable length (e.g., as measured along a longitudinal axis of endovascular device 16) for accessing a target tissue site within the patient from a vascular access point. In examples where endovascular device 16 accesses vasculature in a brain of a patient from a femoral artery access point at the groin of the patient, endovascular device 16 has a length of about 129 centimeters (cm) to about 135 cm, such as about 132 cm, although other lengths may be used.

As described in further detail below, endovascular device 16 may be used to access relatively distal locations in a patient, such as the middle cerebral artery (MCA) in a brain of a patient. The MCA, as well as other vasculature in the brain or other relatively distal tissue sites (e.g., relative to the vascular access point), may be relatively difficult to reach with a catheter (or other elongated body), due at least in part to the tortuous pathway (e.g., comprising relatively sharp twists and/or turns) through the vasculature to reach these tissue sites. Endovascular device 16 may include an elongated body that is structurally configured to be relatively flexible, pushable, and relatively kink- and buckle-resistant, so that it may resist buckling when a pushing force is applied to a relatively proximal portion to advance endovascular device 16 distally through vasculature, and so that it may resist kinking when traversing around a tight turn in the vasculature. Kinking and/or buckling of may hinder a clinician's efforts to push the elongated body distally, e.g., past a turn. In some examples, endovascular device 16 includes a hypotube (e.g., a stainless steel hypotube) positioned proximally of electrodes 17 and expandable element 19. In some examples, endovascular device 16 includes one or more coils (e.g., platinum coils) positioned proximally of electrodes 17 and expandable element 19.

Instead of or in addition to the elongated body of endovascular medical device 16 being configured for intravascular navigation to a cerebral blood vessel to deliver electrical stimulation therapy or sense a patient parameter from a tissue site in brain 18, endovascular medical device 16 can be navigated through vasculature to brain 18 with the aid of a guide member. The guide member can include an outer catheter, an inner catheter, a guide extension catheter, a guidewire, or the like or combination thereof.

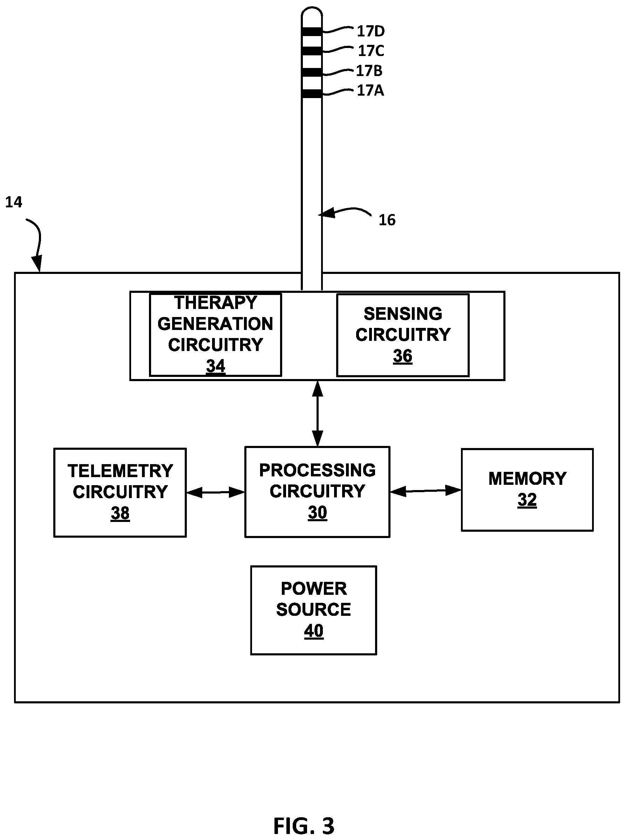

In some examples, endovascular device 16 is a wireless therapy delivery device, such as a microstimulator or the like, which is not electrically coupled to medical device 14 via a wired connection. In some of these wireless therapy delivery device examples, system 10 does not include medical device 14 and endovascular device 16 includes therapy generation circuitry and/or other elements of medical device 14 described herein, e.g., with respect to FIG. 3.

In some examples, more than one endovascular device 16 is positioned within brain 18 of patient 12 to provide stimulation to and/or sense multiple anatomical regions of brain 18. Endovascular device 16 can be implanted in a blood vessel for chronic therapy delivery and/or chronic sensing (e.g., on the order of months or even years) or for more temporary therapy delivery and/or sensing (e.g., on the order of days, such as less than a month or less than 6 months). Temporary therapy delivery may include one or more trial periods, such as to determine, evaluate, or confirm an efficacy of stimulation and/or sensing.

The electrical stimulation therapy described herein (e.g., DBS) may be used to treat various patient conditions, such as, but not limited to, seizure disorders (e.g., epilepsy), pain, migraine headaches, psychiatric disorders (e.g., obsessive compulsive disorder, mood disorders or anxiety disorders), movement disorders (e.g., essential tremor or Parkinson's disease), Huntington's disease, and other neurodegenerative disorders. The anatomic region within brain 18 patient 12 that serve as the target tissue site for electrical stimulation delivered by medical device 14 may be selected based on the patient condition. For example, stimulating an anatomical region, such as the substantia nigra, in brain 18 may reduce the number and magnitude of tremors experienced by patient 12. Other example target anatomical regions for treatment of movement disorders may include the subthalamic nucleus, globus pallidus interna, ventral intermediate, and zona inserta. Anatomical regions such as these may be targeted by the clinician during implantation of endovascular device 16. In other words, the clinician may attempt to position endovascular device 16 within or proximate to these target regions within brain 18 by positioning endovascular device 16 in a cranial blood vessel that is within or proximate to these target regions.

In various examples described herein, example target tissue sites for electrical stimulation delivered via endovascular device 16 positioned in a blood vessel in brain 18 include, but are not limited to, one or more of the anterior thalamus, the ventrolateral thalamus, the subthalamic nucleus (STN), the substantia nigra pars reticulata, the internal segment and/or external segments of the globus pallidus, the ventral intermediate, the zona inserta, the hippocampus (HIP), the dentate gyrus, the cortex (e.g., the motor strip, the sensor strip, the premotor cortex), the fornix, the neostriatum, the ventral intermediate nucleus of the thalamus, the cingulate, or the cingulate gyrus. Example blood vessels that can provide access to these target sites include, but are not limited to, the external carotid, maxillary, or meningeal arteries.

The vasculature into which endovascular device 16 may be inserted and/or guided includes, but is not limited to, veins or arteries. For example, to reach certain deep brain tissue sies, endovascular device 16 can be navigated from a vasculature access site (e.g., in the femoral artery, the radial artery, or another suitable access site) to one or more of the thalamostriate vein, the internal cerebral vein, the basal vein of Rosenthal, the inferior/superior sagittal sinus, the anterior choroidal artery, or any related combinations thereof.

Certain intracranial blood vessels into which endovascular device 16 may be inserted and/or guided may be located at different distances from different target tissue sites. Such distances may play a role in efficacy of therapy delivered by endovascular device 16, as a closer distance may indicate a shorter distance any electrical stimulation signal may have to travel, and, in some examples, the less power that is needed to generate an efficacious electrical stimulation signal. For example, the thalamostriate vein may be approximately 1.2 millimeters (mm) in diameter and be located approximately 0-2 mm from the anterior nucleus of the thalamus (ANT) and 0-2 mm from the fornix. As another example, the internal cerebral vein may be 1.9 mm plus or minus up to 0.5 mm in diameter and be located approximately 5-10 mm from the ANT and approximately 2-5 mm from the fornix. The basal vein of Rosenthal may be 1.7 mm plus or minus up to 0.4 mm in diameter and be located approximately 10-15 mm from the ANT, approximately 5-10 mm from the HIP, and approximately 5-10 mm from the STN. The inferior sagittal sinus may be 1.3 mm plus or minus up to 0.3 mm in diameter and be located approximately 10-15 mm from the Fornix.

A clinician can also select a particular intracranial blood vessel to position electrodes 17 at different orientations or distances relative to tissue sites for which it may be desirable to avoid electrical stimulation to minimize or even eliminate adverse effects. DBS may cause one or more side effects by inadvertently providing electrical stimulation to anatomical regions near the targeted anatomical region. For at this reason, a clinician may position electrodes 17 within brain 18 and/or program the stimulation parameters in order to balance effective therapy and minimal side effects.

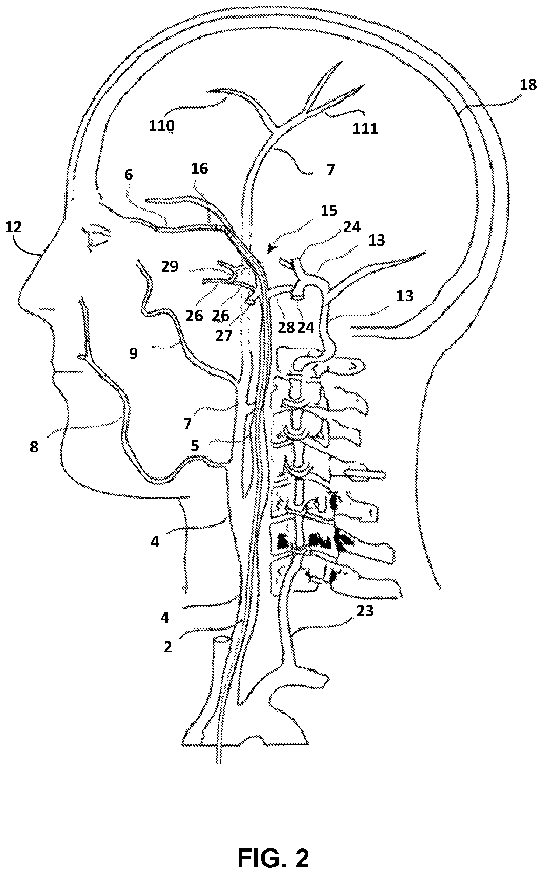

As discussed in further detail below with reference to FIG. 2, in some examples, endovascular device 16 is configured to be delivered to one or more target sites in brain 18 via vasculature of patient 12. Thus, rather than introducing endovascular device 16 into brain tissue (e.g., the cerebral parenchyma) via a burr hole through a skull of patient 12 or the like, endovascular device 16 is configured to be navigated proximate to a target electrical stimulation site in brain 18 via vasculature of patient 12. The endovascular delivery of endovascular device 16 to deep brain sites in brain 18 can help minimize the invasiveness of therapy system 10.

As discussed below, electrodes 17 can be positioned on an expandable element 19 of endovascular device 16 that is configured to expand radially outwards from a relatively low profile delivery configuration to a deployed configuration. This may enable electrodes 17 to be held in apposition with a blood vessel wall, promote tissue ingrowth around electrodes 17 along the vessel wall (while still leaving a patent lumen to enable blood flow through the blood vessel despite implantation of endovascular device 16), which can reduce the overall power needed to deliver efficacious electrical stimulation therapy to a target tissue site of brain 18, and help secure electrodes 17 in place in the blood vessel for chronic therapy delivery.

Medical device 14 can be an external medical device or an implantable medical device that includes electrical stimulation circuitry configured to generate and deliver electrical stimulation therapy to patient 12 and/or sensing circuitry configured to sense a patient parameter (e.g., a physiological signal) via one or more electrodes 17 of endovascular device 16. In the example shown in FIG. 1, endovascular device 16 is coupled to medical device 14 via a connector 22, which defines a plurality of electrical contacts for electrically coupling electrodes 17 to electrical stimulation generation circuitry and/or sensing circuitry within medical device 14. Connector 22 may also be referred to as a connector block or header of medical device 14. In the example of FIG. 1, endovascular device 16 is indirectly coupled to connector 22 with the aid of lead extension 24. In some examples, endovascular device 16 is directly coupled to connector 22 without the aid of extension 24.

In some examples, medical device 14 is configured to be implanted in patient 12 in any suitable location, such as a location outside of brain 18, e.g., in a pectoral region. In other examples, medical device 14 is configured to be external to patient 12. Endovascular device 16 may be, for example, implanted within a cranial vein and one or more proximal wires/leads can remain within the venous system until they exit the venous system, such as through the subclavian vein in the chest or the internal jugular vein in the neck for implant in the pectoral region. In yet other examples, some or all of medical device 14 is configured to be implanted in brain 18, e.g., as part of endovascular device 16.

As shown in FIG. 1, system 10 may also include a programmer 20, which may be a handheld device, portable computer, or workstation that provides a user interface to a user, for example a clinician or other user, such as a patient. The user may interact with the user interface to program electrical stimulation parameters for medical device 14.

With the aid of programmer 20 or another computing device, a clinician may select values for therapy parameters for controlling therapy delivery by therapy system 10. The values for the therapy parameters may be organized into a group of parameter values referred to as a "therapy program" or "therapy parameter set." "Therapy program" and "therapy parameter set" are used interchangeably herein. In the case of electrical stimulation, the therapy parameters may include an electrode combination, a power, and an amplitude, which may be a current or voltage amplitude, and, if medical device 14 delivers electrical pulses, a pulse width, and a pulse rate for stimulation signals to be delivered to the patient. Other example therapy parameters include a slew rate, duty cycle, and phase of the electrical stimulation signal.

An electrode combination may include a selected subset of one or more electrodes 17 located on one or more implantable endovascular device 16 coupled to medical device 14. The electrode combination may also refer to the polarities of the electrodes in the selected subset. By selecting particular electrode combinations, a user may target particular anatomic structures within brain 18 of patient 12. In addition, by selecting values for slew rate, duty cycle, phase amplitude, pulse width, and/or pulse rate, the user can attempt to generate an efficacious therapy for patient 12 that is delivered via the selected electrode subset.

Whether programmer 20 is configured for clinician or patient use, programmer 20 may communicate with medical device 14 or any other computing device via wireless or a wired communication. Programmer 20, for example, may communicate via wireless communication with medical device 14 using radio frequency (RF) telemetry techniques. Programmer 20 may also communicate with another programmer or computing device via a wired or wireless connection using any of a variety of local wireless communication techniques, such as RF communication according to the 802.11 or Bluetooth specification sets, infrared communication according to the Infrared Data Association (IRDA) specification set, or other standard or proprietary telemetry protocols. Programmer 20 may also communicate with another programming or computing device via a wired or wireless communication technique.

In some examples, in addition to or instead of delivering electrical stimulation to brain 18, endovascular medical device 16 can also be used to sense one or more patient parameters, such as bioelectrical signals, either using electrodes 17 or other types of sensors that are carried by endovascular medical device 16. In some examples, the bioelectrical signals sensed within brain 18 reflect changes in electrical current produced by the sum of electrical potential differences across brain tissue. Examples of bioelectrical brain signals that can be sensed via one or more electrodes of electrodes 17 include, but are not limited to, electrical signals generated from local field potentials within one or more regions of brain 18, an EEG signal, an electrocorticogram (ECoG) signal, or an evoked potential. In some examples, a sensing parameter includes one or more of a direction faced by each sensing electrode or group of electrodes (e.g., the active electrodes with which a medical device senses a patient parameter), a location of one or more electrodes within brain 18, or other parameters that may affect detection and/or sensing of one or more patient parameters.

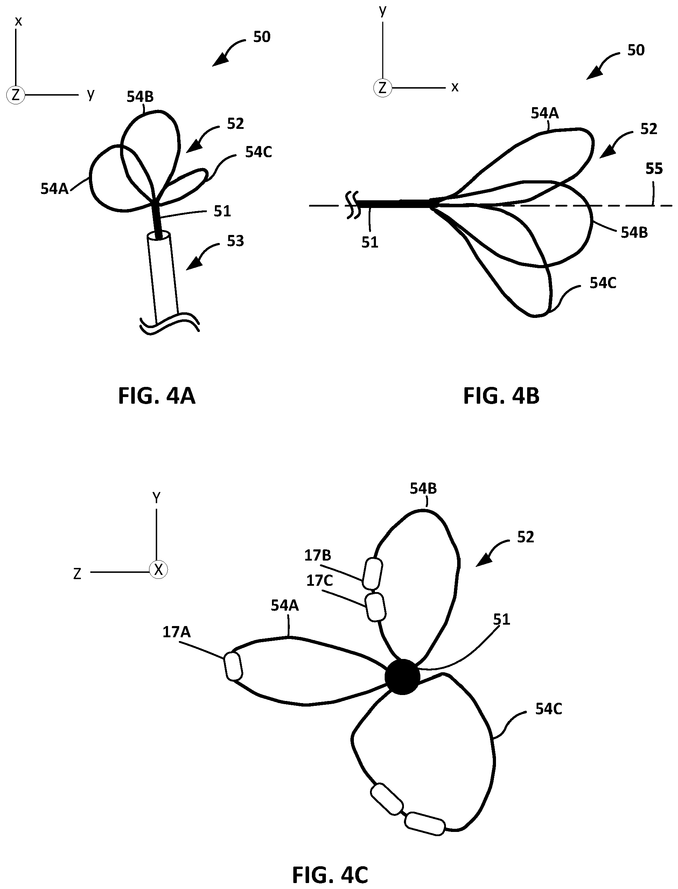

Endovascular device 16 may have any suitable configuration for delivering electrical stimulation to a target tissue site in brain 18 or sensing a patient parameter from an endovascular location (e.g., in a cranial vein or a cranial artery). For example, endovascular device 16 can define or otherwise include one or more loops, examples of which are described in further detail with respect to FIGS. 4A-6B. Thus, one or more loops are configured to expand radially outwards to place electrodes 17 in contact with a vessel wall and enable electrodes 17 to obtain better wall acquisition for reduction of thrombus generation. In some examples, endovascular device 16 further includes a helical section, a linear loop, a clustered section, a braided section, a stent or any related combination thereof. In some examples, one or more electrodes 17 are configured to deliver directional stimulation and/or directional sensing (e.g., electrodes 17 can include segmented or partial ring electrodes) or be positioned on device 16 to provide electrical stimulation and/or directional sensing in a direction less than 360 degrees around a longitudinal axis of endovascular device 16).

In some examples, endovascular device 16 includes or otherwise is coupled to one or more electrical leads which may be further connected to medical device 14 or may be wirelessly connected to endovascular device 16.

Each of one or more of electrodes 17 of endovascular device 16 may be disposed at a respective location on endovascular device 16. In some examples, the respective location includes a distal portion, a central portion, or a proximal portion of endovascular device 16. In some examples, the respective location includes a circumferential location anywhere along the length of endovascular device 16 or on radiopaque makers of endovascular device 16, including coils, marker bands, or any related combination of locations thereof. In some examples, the respective location of each electrode of electrodes 17 is configured for directional stimulation and/or directional sensing. In some examples, a number of electrodes 17 is anywhere between 1 and 50, inclusive. In some examples, a number of lead wires from endovascular device 16 is between 1 and 50, inclusive.

Individual electrodes 17 or a group of two or more electrodes 17 can have any suitable shape. As an example, a shape of individual or combined electrodes of electrodes 17 may include square, rectangle, circular, spherical, oval, cubic, ovoid, cuboid, or others. A surface area of individual or combined electrodes of electrodes 17 may vary from 0.01 square millimeters (mm²) to 200 square millimeters, inclusive.

Medical device 14 is configured to generate and deliver any suitable electrical stimulation signal, which can be a continuous time signal (e.g., a sinusoidal waveform or the like) or a plurality of pulses. In some examples, the electrical stimulation waveform generated by medical device 14 and which be delivered by one or more of electrodes 17 is a charge balanced, biphasic waveform. In some examples, such an electrical stimulation waveform consists of periodic pulses or otherwise include periodic pulses, or can include a continuous time waveform.

In some examples, endovascular device 16 includes at least one expandable element 19 configured to expand radially outwards, and one or more electrodes 17 are positioned on (e.g., coupled to, defined by, or otherwise carried by) expandable element 19. In some examples, one or more sensors that are different from electrodes 17 can also be positioned on the same expandable element (e.g., expandable element 19) as one or more electrodes 17 or on a different expandable element (e.g., expandable element 19) of endovascular device 16. Expandable element 19 can have any suitable configuration that enables endovascular device 16 to assume a relatively low-profile configuration (also referred to herein as a "delivery" or "compressed" configuration in some examples) to facilitate delivery through vasculature to a target tissue site in brain 18 and expand radially outwards (relative to a central longitudinal axis of endovascular device 16) to position the one or more electrodes 17 closer to target brain tissue (also referred to herein as an "expanded" or "deployed" configuration in some examples).

In some examples, expandable element 19 is configured to expand radially outwards with sufficient force and to a cross-sectional dimension (e.g., a diameter) sufficient to position the one or more electrodes 17 in apposition with a blood vessel wall. Positioning one or more electrodes 17 in apposition with a blood vessel wall may help promote tissue ingrowth around electrodes 17, which can reduce the impedance and the overall power needed to deliver efficacious electrical stimulation therapy to a target tissue site of brain 18, and help secure electrodes 17 in place in the blood vessel for chronic (e.g., on the order of months or even years) therapy delivery. Fixing endovascular device 16 in place within the blood vessel via the tissue ingrowth or, in some examples, using another fixation structures/anchoring mechanisms, such as tines, coils barbs, or the like, can also help reduce the possibility of thrombosis.

Expandable element 19 can be configured to expand radially outwards using any suitable technique. In some examples, expandable element 19 includes a shape memory (e.g., nitinol) material that enables the expandable element to assume a predetermined shape in the absence of a force (e.g., a compressive or tensile force) holding expandable element 19 in a relatively low-profile delivery configuration. For example, expandable element 19 can be configured to expand radially outwards upon deployment from an outer sheath (e.g., an outer catheter), or upon the proximal withdrawal of a straightening element (e.g., a guidewire or a mandrel) positioned in an inner lumen of the endovascular device 16. In some examples, expandable element 19 is configured to expand radially outwards in response to proximal withdrawal of a pull member attached to a distal portion of the endovascular device 16 or in response to a distal movement of an elongated control member attached to the expandable element.

Expandable element 19 can have any suitable configuration in its deployed (e.g., expanded) configuration. For example, expandable element 19 can be a basket, include one or more splines or arms configured to expand radially outwards, define one or more loops, define a helical or spiral element, or the like or combinations thereof, when in the deployed configuration. One or more expandable elements 19 may be disposed at various positions along endovascular device 16 (e.g., at one or more longitudinal positions along endovascular device 16).

In addition to, or instead of, chronic therapy delivery and/or chronic sensing, example devices, systems, and methods described herein can be used for more temporary applications. In some examples, a first endovascular device (e.g., configured like endovascular device 16 or having another configuration) is configured to be operated in an acute (e.g., temporary) trial mode for a trial period to determine, evaluate, or confirm an efficacy of stimulation and/or sensing. For example, endovascular device 16 (including electrodes 17, medical device 14, processing circuitry, etc.) may be configured to operate in the trial mode to determine the efficacy of one or more stimulation parameter values and/or one or more sensing parameters. After the acute trial period, the first endovascular device may be removed, and a second endovascular device (e.g., configured like endovascular device 16 or having another configuration) configured to operate in a chronic mode may be implanted for a chronic period for chronic (e.g., long term, or permanent) stimulation therapy or sensing. In some examples, a first endovascular device (e.g., for use in the acute trial mode) is configured to be implanted and subsequently removed after the trial period.

A trial period has a shorter intended duration than a chronic period, though the ultimate length of the chronic period may be less than an intended duration due to one or more factors, such as a patient response that requires shortening the chronic period relative to the intended duration of the chronic period. In some examples, the trial period includes a trial period length on the order of minutes (e.g., 1 minute, 2 minutes, 3 minutes, 5 minutes, 30 minutes, 45 minutes, etc.), on the order of hours (e.g., 1 hour, 2 hours, 5 hours, 12 hours, etc.), on the order of days (e.g., 1 day, 2 days, 3 days, etc.), on the order of weeks (e.g., 1 week, 2 weeks, 3 weeks, etc.) on the order of months (e.g., 1 month, 2 months, 3 months, etc.), or longer. In some examples, one or more endovascular devices may be used for multiple trial periods (e.g., successive trial periods) for determining an efficacy of one or more stimulation parameters and/or one or more sensing parameters.

In some examples, the trial period length is selected based on an endothelization profile. The endothelization profile may include a length of time for at least part of an endovascular device to become incorporated (or at least partially incorporated) into a vessel wall via tissue ingrowth or the like. For example, the length of the trial period may be selected such that the trial period is shorter than a period for an endovascular device (e.g., endovascular device 16) to become incorporated into a vessel wall via endothelization. The length of the trial period may be selected such that trial period is shorter than a period for the endovascular device to reach a threshold level of endothelization. The length of the trial period may correspond to a predetermined profile of endothelization (e.g., based on a model of endothelization, which can be patient specific or more general to a population of patients).

In some examples, the first endovascular device (e.g., for use in the acute trial mode) does not include features (e.g., surface features) to promote endothelization. This may enable a clinician to remove the first endovascular device after the trial period with minimal impacts to a vessel wall (e.g., a portion of a vessel wall adjacent the endovascular device).

In some examples, the first endovascular device (e.g., the endovascular device used for the trial period) is configured to transform from a deployed configuration to a relatively low-profile configuration (as described above) to facilitate removal from a blood vessel of patient 12 after the trial period. For example, the first endovascular device (e.g., endovascular device 16) may be configured to recaptured, re-sheathed, or otherwise retracted (e.g., into a catheter or other outer sheath) to facilitate removal from patient 12 after the trial period. In examples in which the endovascular device includes the expandable element (as described in connection with later figures), the expandable element may be collapsed (e.g., via an outer sheath, pullwire, or another suitable method) to facilitate removal of the endovascular device from the vasculature of patient 12.

After determining or confirming the efficacy (e.g., the efficacy of one or more stimulation parameters and/or one or more sensing parameters) of the first endovascular device during the trial period, a second endovascular device (e.g., endovascular device 16) may be implanted and configured to operate in the chronic mode. In some examples, the second endovascular device is configured to deliver stimulation or sense a patient parameter in the chronic mode based on feedback from the trial period. For example, one or more therapy parameter values, as described above, are selected and/or changed based on feedback (e.g., one or more sensed patient parameters, feedback from patient 12 regarding the effects of therapy, and/or other indicators of the efficacy of therapy delivery) from the trial period.

As another example, one or more sensing parameters is selected and/or changed for the chronic period based on feedback from the trial period. In some examples, one or more implant locations within the brain is selected based on feedback from the trial period. In some examples, an orientation (e.g., a direction of one or more electrodes 17 facing outward from endovascular device 16) of the endovascular device is selected based on feedback from the trial period. In some examples, the second endovascular device includes one or more deployable configurations, surface textures or coatings, as described above, to promote endothelization around at least a portion of the second endovascular device.

While the second endovascular device may be provided for chronic therapy delivery or sensing, the first endovascular device (e.g., the endovascular device used for the trial period) may also be used during the chronic period. In some examples, a second endovascular device is not provided for the chronic period if the first endovascular device is used for the chronic period. In some examples, the first endovascular device is moved or repositioned for the chronic period. For example, endovascular device 16 may be moved or reposition within the vasculature of patient 12 for the chronic period. The first endovascular device may be configured to operate in the chronic mode.

In some examples, the first endovascular device (e.g., the endovascular device used for the trial period) and the second endovascular device (e.g., the endovascular device used for the chronic period) may have different configurations of leads, electrodes, or operating parameters. As an example, the first endovascular device may include more electrodes as compared to the second endovascular device, which can provide more granular sensing or stimulation to enable a clinician to better identify therapy and/or sensing targets in brain 18. In some examples, the first endovascular device includes fewer electrodes than the second endovascular device. However, in some examples, the first endovascular device includes the same number of electrodes as compared to the second endovascular device.

In some examples, the same medical device 14 (e.g., including an electrical stimulation generation circuitry and/or sensing circuitry) is used for both the trial and chronic periods. In other examples, different medical devices are used for the trial and chronic periods. For example, the first endovascular device may be configured to electrically and mechanically connect to an external medical device configured to generate stimulation therapy and/or sense a patient parameter (e.g., bioelectrical brain signals) via electrodes of the first endovascular device and the second endovascular device may include a medical device (e.g., medical device 14) configured to be implanted in patient 12. In some examples, the external medical device can be configured similarly to medical device 14, but may not be configured for implantation in patient 12. In other examples, the external medical device can include fewer features than medical device 14, e.g., less memory, less sophisticated control circuitry. In addition, in some examples, the external medical device can be pre-programmed (e.g., by a clinician or a manufacturer) to deliver electrical stimulation therapy according to only a preselected group of therapy programs. In contrast, in some cases, a clinician programs medical device 14 used for chronic therapy delivery to deliver efficacious electrical stimulation therapy to patient 12 or effective sensing of one or more parameters of patient 12 during a programming period.

FIG. 2 shows example arterial vasculature of brain 18 of patient 12. Brain 18 is supplied with blood through the carotid and the vertebral arteries on each side of the neck. The arteries include the common carotid artery 4 in the neck, which is a common access pathway for the various devices and/or methods disclosed herein, the internal carotid 5 which supplies the ophthalmic artery 6. The external carotid 7 supplies the maxillary artery 8, the middle meningeal artery 9, and the superficial temporal arteries 110 (frontal) and 111 (parietal). The vertebral artery 23 supplies the basilar artery 13 and the cerebral arteries including the posterior cerebral artery 24 and the circle of Willis indicated generally at 15. The siphon of the vertebral artery appears in the intracranial vasculature on the vertebral approach to the Circle of Willis. Also supplied by the internal carotid artery are the anterior cerebral artery 26 and the MCA 27, as well as the circle of Willis, including the posterior communicating artery 28 and the anterior communicating artery 29. The siphon of the internal carotid artery 5 appears in the intracranial vasculature on the carotid approach into the Circle of Willis. These arteries can have an internal diameter of about 1 mm to 5 mm, such as from 2-4 mm.

The devices, systems, and methods described herein enable delivery of endovascular device 16 to tissue sites in brain 18 via these arteries for treatment of the tissue sites in brain 18. Endovascular device 16 can be navigated to the cranial vasculature to reach the deep brain tissue sites, e.g., via an insertion catheter 2 (which can comprise, for example, a microcatheter) is shown extending through the common carotid artery 4 and the internal carotid artery 5, with endovascular device 16 extending through the catheter 2 and into the anterior cerebral artery 26.

In some examples, endovascular device 16 is navigated through the vasculature over catheter 2, alone or with the aid of a guidewire. In other examples, endovascular device 16 is navigated through the vasculature through an inner lumen of catheter 2, alone or with the aid of a guidewire. In some examples, catheter 2 is configured to be advanced through the vasculature from a pushing force applied to a proximal portion of catheter 2, without buckling or undesirable bending (e.g., kinking) of the catheter body. In some examples, the flexible catheter body of catheter 2 is configured to substantially conform to the curvature of the vasculature. In addition, in some examples, the catheter body of catheter 2 has a columnar strength and flexibility that allow at least a distal portion of the catheter body to be navigated from a femoral artery, through the aorta of the patient, and into the intracranial vascular system of patient 12, e.g., to reach a relatively distal treatment site, including MCA 27, the Circle of Willis, and tissue sites more distal than the MCA 27 and the Circle of Willis. The MCA 27 and, consequently, vasculature distal to the MCA 27 may be relatively difficult to access due to the carotid siphon or vertebral artery anatomy that must be traversed to reach such locations.

The devices, systems, and methods described herein enable delivery of endovascular device 16 to tissue sites in brain 18 via venous structures as well, including but not limited to, the internal jugular vein and any of the sigmoid/transverse/straight, superior sagittal/inferior sagittal sinuses, for treatment of the tissue sites in brain 18.

FIG. 3 is a functional block diagram illustrating components of an example medical device 14, which is configured to generate and deliver electrical stimulation therapy to patient 12 and, in some examples, sense one or more patient parameters, such as bioelectrical brain signals or other physiological parameter of patient 12. Medical device 14 includes processing circuitry 30, memory 32, therapy generation circuitry 34, sensing circuitry 36, telemetry circuitry 38, and power source 40.

Therapy generation circuitry 34 includes any suitable configuration (e.g., hardware) configured to generate electrical stimulation signals to a target tissue site in brain 18 of patient 12. Processing circuitry 30 is configured to control therapy generation circuitry 34 to generate and deliver electrical stimulation therapy via electrodes 17 (shown individually as electrode 17A, electrode 17B, electrode 17C, and electrode 17D in the example of FIG. 3) of endovascular deice 16. The parameter values may be selected based on the patient condition being addressed, as well as the target tissue site in brain 18 for the electrical stimulation therapy. The electrical stimulation therapy can be provided via stimulation signals of any suitable form, such of stimulation pulses or continuous-time signals (e.g., sine waves).

Sensing circuitry 36 is configured to sense a physiological parameter of a patient. Sensing circuitry 36 may include any sensing hardware configured to sense a physiological parameter of a patient, such as, but not limited to, one or more electrodes, optical receivers, pressure sensors, or the like. The one or more sensing electrodes can be the same or different from electrodes 17 configured to deliver electrical stimulation therapy. Processing circuitry 30 can use the sensed physiological signals to control therapy delivery by therapy generation circuitry 34, e.g., the timing of the therapy delivery or one or more characteristics of the electrical simulation signal generated by therapy generation circuitry 34.

In some examples, sensing circuitry 36 is configured to sense a bioelectrical brain signal via one or more electrodes 17 (e.g., all or a subset of electrodes 17). Thus, electrodes 17 can be configured to receive or transmit energy (e.g., current). Example bioelectrical brain signals include an EEG signal, an ECoG signal, a signal generated from measured field potentials within one or more regions of brain 18, action potentials from single cells within brain 18 (referred to as "spikes"), or evoked potentials. Determining action potentials of single cells within brain 18 may require resolution of bioelectrical signals to the cellular level and provides fidelity for fine movements, i.e., a bioelectrical signal indicative of fine movements (e.g., slight movement of a finger). In examples in which endovascular medical device 16 is configured to sense an evoked potential, endovascular medical device 16 may also be configured to generate a stimulus (e.g., via therapy generation circuitry 34, alone or in combination with processing circuitry 30) to elicit the evoked potential. For example, endovascular device 16 can generate and deliver electrical stimulation to tissue in brain 18 and sense an evoked compound action potential (ECAP). An ECAP is synchronous firing of a population of neurons which occurs in response to the application of a stimulus including, in some cases, an electrical stimulus by endovascular device 16. The ECAP may be detectable as being a separate event from the stimulus itself, and the ECAP may reveal characteristics of the effect of the stimulus on the tissue.

In some examples, processing circuitry 30, alone or in combination with sensing circuitry 36, is configured to determine a level of endothelization proximate one or more of the electrodes 17 based on a sensed parameter. The parameter is any suitable parameter that can be sensed by processing circuitry 30 via electrodes 17 or another sensor and that changes as a function of the level of endothelization and can include, for example, impedance of an electrical pathway that includes the one or more electrodes 17. In some examples, a level of endothelization proximate electrodes 17 includes the extent to which endothelial cells cover endovascular device 16 and/or one or more electrodes 17, which may affect the impedance of the respective electrodes 17. The level can include a level at a particular point in time or a change in a level of endothelization over time, e.g., relative to a previously determined level, such as a baseline level at the time of implantation of endovascular device 16 in patient 12. For example, the change in a level of endothelization (e.g., an altered impedance) can be a result of sensing a level of impedance of one or more electrodes of electrodes 17 at a first time point and at a second time point, and calculating a change in the impedance level between the first time point and the second time point.

In some examples, processing circuitry 30, alone or in combination with therapy generation circuitry 34, is configured to select one or more electrical stimulation parameter values based on a determined level of endothelization or in response to detecting a change in the level of endothelization proximate electrodes 17. As described above, the electrical stimulation parameters (or "therapy parameters") may include an electrode combination (e.g., selective activation of a group or subset of electrodes from electrodes 17), a power, and an amplitude, which may be a current or voltage amplitude, and, if medical device 14 delivers electrical pulses, a pulse width, and a pulse rate or frequency for stimulation signals to be delivered to the patient. Other example electrical stimulation parameters include a slew rate, duty cycle, and phase of the electrical stimulation signal. In some examples, processing circuitry 30, alone or in combination with therapy generation circuitry 34, is configured to automatically select or adjust one or more electrical stimulation parameter values (e.g., without clinician intervention) or may otherwise provide a prompt or recommendation to select or adjust one or more electrical stimulation parameter values (e.g., such as via programmer 20). In some examples, a sensed level of endothelization is utilized along with patient factors (e.g., symptoms, side effects, or other sensed parameters) to select or adjust one or more stimulation parameters. Endothelization proximate endovascular device 16 and or electrodes 17 may facilitate the ability to provide stimulation therapy or sense patient parameters, which may enhance the fidelity of therapy. In some examples, when a sufficient level of endothelization has been reached, a need for anticoagulation therapy (e.g., commonly dual antiplatelet therapy (DAPT)) is reduced or eliminated.

In some examples, some electrodes 17 can be shorted together and/or some electrodes 17 can be individually electrically connected to therapy generation circuitry 34 and/or sensing circuitry 36 to enable selective activation of electrodes 17 as needed, e.g., to achieve direction stimulation or sensing. Shorting some or all of electrodes 17 (including groups or subsets of shorted electrodes 17) may include connecting electrodes via a common conductor, such that all shorted electrodes may be controlled together for delivering electrical stimulation and or sensing. In some examples, each electrode 17 is individually electrically connected to therapy generation circuitry 34 and/or sensing circuitry 36, such that each electrode 17 can be selectively activated by processing circuitry 30.

In some examples, sensing circuitry 36 and/or processing circuitry 30 includes signal processing circuitry configured to perform any suitable analog conditioning of the sensed physiological signals. For example, sensing circuitry 36 may communicate to processing circuitry 30 an unaltered (e.g., raw) signal. Processing circuitry 30 may be configured to modify a raw signal to a usable signal by, for example, filtering (e.g., low pass, high pass, band pass, notch, or any other suitable filtering), amplifying, performing an operation on the received signal (e.g., taking a derivative, averaging), performing any other suitable signal conditioning (e.g., converting a current signal to a voltage signal), or any combination thereof. In some examples, the conditioned analog signals are processed by an analog-to-digital converter of processing circuitry 30 or other component to convert the conditioned analog signals into digital signals. In some examples, processing circuitry 30 operates on the analog or digital form of the signals to separate out different components of the signals. In some examples, sensing circuitry 36 and/or processing circuitry 30 performs any suitable digital conditioning of the converted digital signals, such as low pass, high pass, band pass, notch, averaging, or any other suitable filtering, amplifying, performing an operation on the signal, performing any other suitable digital conditioning, or any combination thereof. Additionally or alternatively, sensing circuitry 36 may include signal processing circuitry to modify one or more raw signals and communicate to processing circuitry 30 one or more modified signals.

In some examples, processing circuitry 30, alone or in combination with therapy generation circuitry 34 and/or sensing circuitry 36, is configured to operate medical device 14 (including electrodes 17, endovascular device 16, etc.) in a trial mode for a trial period to determine an efficacy of electrical stimulation or sensing. As described above, a trial mode can include a trial period of stimulation and/or sensing to determine, evaluate, or confirm an efficacy of stimulation and/or sensing. In some examples, processing circuitry 30, alone or in combination with therapy generation circuitry 34 and/or sensing circuitry 36, is configured to deliver electrical stimulation therapy and/or sense a patient parameter during the trial period. In some examples, processing circuitry 30 is configured to determine, evaluate, or confirm an efficacy of stimulation and/or sensing. For example, processing circuitry 30 may determine one or more therapy parameters for chronic stimulation and/or sensing based on the trial period.

Although shown as part of medical device 14 in FIG. 3, in other examples, sensing circuitry 36 can be a part of a device separate from medical device 14. For example, sensing circuitry 36 can be part of an implantable sensing device implanted in cranial vasculature or elsewhere in brain 18 of patient 12.

Processing circuitry 30, as well as other processors, processing circuitry, controllers, control circuitry, and the like, described herein, may include any combination of integrated circuitry, discrete logic circuity, analog circuitry, such as one or more microprocessors, digital signal processors (DSPs), application specific integrated circuits (ASICs), or field-programmable gate arrays (FPGAs). In some examples, processing circuitry 30 includes multiple components, such as any combination of one or more microprocessors, one or more DSPs, one or more ASICs, or one or more FPGAs, as well as other discrete or integrated logic circuitry, and/or analog circuitry.

Memory 32 is configured to store program instructions, such as software, which may include one or more program modules, which are executable by processing circuitry 30. When executed by processing circuitry 30, such program instructions may cause processing circuitry 30 to provide the functionality ascribed to processing circuitry 30 herein. The program instructions may be embodied in software and/or firmware. Memory 32 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

Processing circuitry 30 is configured to control telemetry circuitry 38 to send and receive information. Telemetry circuitry 38, as well as telemetry modules in other devices described herein, such as programmer 20 (FIG. 1), may accomplish communication by any suitable communication techniques, such as RF communication techniques. In addition, telemetry circuitry 38 may communicate with external medical device programmer 20 via proximal inductive interaction of medical device 14 with programmer 20. Accordingly, telemetry circuitry 27 may send information to external programmer 20 on a continuous basis, at periodic intervals, or upon request from medical device 14 or programmer 20.

Power source 40 is configured to deliver operating power to various components of medical device 14. Power source 40 may include a small rechargeable or non-rechargeable battery and a power generation circuit to produce the operating power. Recharging may be accomplished through proximal inductive interaction between an external charger and an inductive charging coil within medical device 14. In some examples, power requirements may be small enough to allow medical device 14 to utilize patient motion and implement a kinetic energy-scavenging device to trickle charge a rechargeable battery. In other examples, traditional batteries may be used for a limited period of time.

As discussed elsewhere, in some examples, endovascular device 16 is configured to be a standalone electrical stimulation device and can include one or more elements of medical device 14 shown in FIG. 3.

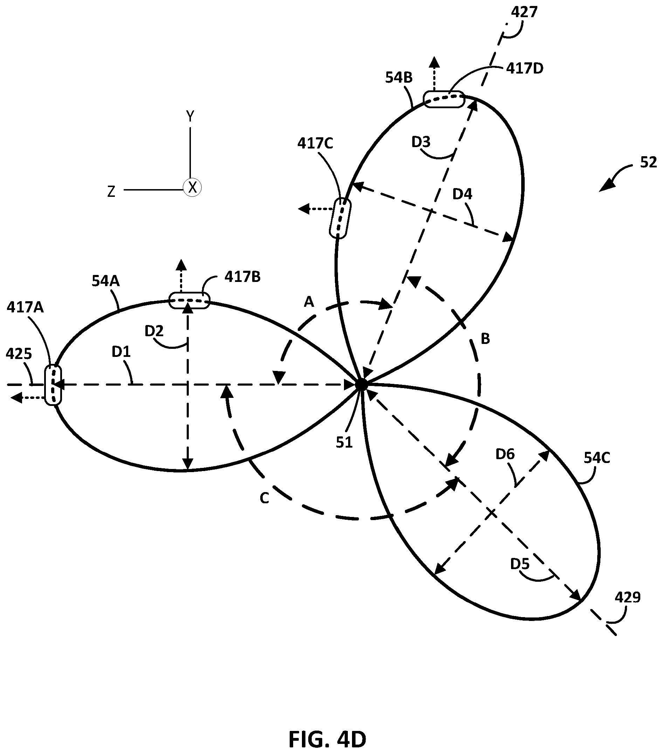

FIGS. 4A-4D illustrate different views of an example endovascular device 50, which is an example of endovascular device 16. FIGS. 4A and 4B illustrate side views of endovascular device 50 (e.g., a view parallel to a x-y plane, where orthogonal x-y-z axes are shown in the figures for ease of description), and FIGS. 4C and 4D illustrate end view of endovascular device 50 (i.e., a view parallel to a y-z plane). Endovascular device 50 includes an expandable element 52 (which may be an example of expandable element 19 of FIG. 1). Expandable element 52 is configured to expand radially outwards from a relatively low-profile (e.g., linear) configuration to the configuration shown in FIGS. 4A-4D, which is an example of a deployed configuration. Expandable element 52 is shown as expanding radially outwards in response to deployment from a lumen of an outer sheath 53. Endovascular device 50 can include an elongated body 51 (e.g., which may be disposed within and extend distally from outer sheath 53, as shown in FIG. 4A) connected to expandable element 52. For example, expandable element 52 can be part of elongated body 51 or separate from and attached to a distal portion or another portion of elongated body 51. Thus, although FIGS. 4A-4D illustrate an example in which expandable element 52 is at a distalmost end of elongated body 51, in other examples, expandable element 52 can additional or alternatively be positioned proximal to the distal end of elongated body 51.

Expandable element 52 includes radially expanding loops 54A, 54B, and 54C. Loops 54A-54C can define any suitable open (e.g., partial) or closed loop and can be circular, non-circular, or the like. Loops 54A-54C can have, for example, a flower petal shape when expanded. The flower petal shape may include one or more of an oval, obovate, ovate, and round flower petal shape. For example, an obovate petal shape includes a flower petal shape with a base (e.g., a side with a connection point and/or a side closest to elongated body 51) that narrows relative to an end opposite the base. Loops 54A-54C are shown as expanding radially outwards from a common location (e.g., an outer surface or a distal end of an elongated body), e.g., to define a flower-like shape. In other examples, loops 54A-54C may not converge to a common location.

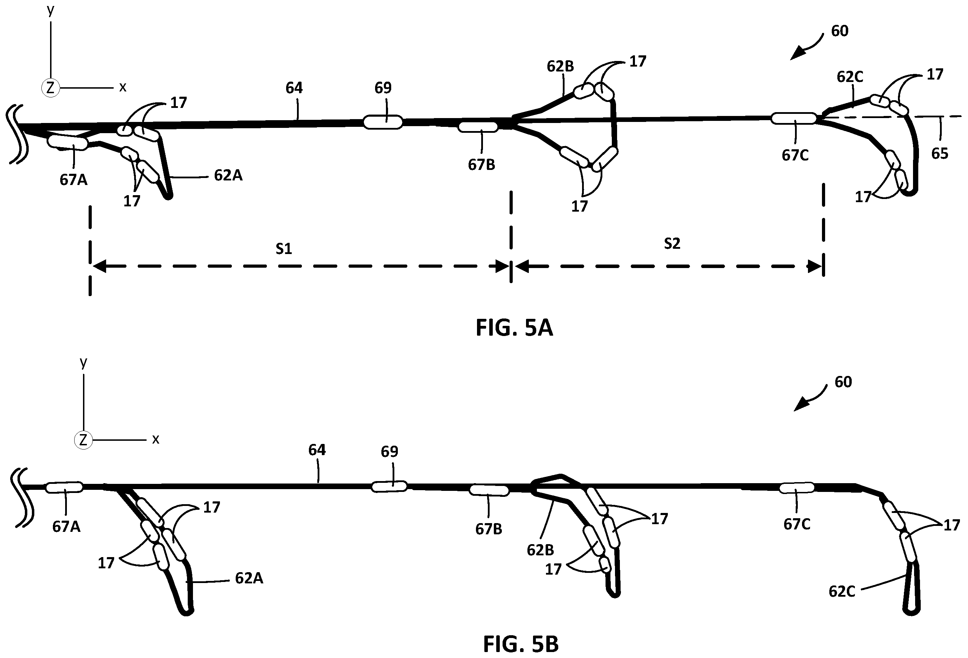

Although three loops 54A-54C are shown in FIGS. 4A-4C, in other examples, expandable element 52 can have one loop, two loops, or more than three loops. Further, although three loops 54A-54C are shown at a distal portion of elongated body 51, loops can be disposed at more proximal location from the distal portion of elongated body 51. In some examples, endovascular device 50 includes multiple sets of loops (such as loops 54A-54C) disposed at locations spaced longitudinally apart along elongated body 51. An example of longitudinally spaced loops is shown with respect to FIGS. 5A and 5B. Although FIGS. 5A and 5B illustrate only one loop at each longitudinal location, in other examples, one or more (e.g., all or less than all) of the longitudinal locations of expandable elements can include a plurality of loops, e.g., each having a flower petal shape.

In the example of FIG. 4C, electrodes 17 (e.g., labeled individually as electrode 17A, electrode 17B, and electrode 17C, and also referred to as "one or more electrodes 17") are positioned on loops 54A and 54B. Some or all loops 54A-54C can have one or more electrode 17, which are configured to deliver electrical stimulation signals (e.g., in a monopolar or bipolar arrangement) and/or sense a patient parameter. In some examples, each loop 54A-54C has only one electrode. In other examples, each loop 54A-54C has two or more electrodes. Electrodes 17 can have any suitable position on the loops 54A-54C. For example, electrode 17A is shown at a distal-most end of loop 54A, while electrodes 17B, 17C are shown as being between the base and the end of the loop opposite the base. The electrode locations can be selected to provide the desired level of contact with a blood vessel wall and/or to provide the desired level of directional sensing or directional stimulation.

In some examples, in addition to or instead of having electrodes 17 positioned on one or more loops 54A-54C, one or more of the loops themselves define an electrode. For example, a loop 54A, 54B, and/or 54C can be formed from an electrically conductive material that is exposed to define an electrode configured to deliver electrical stimulation therapy and/or sense a bioelectrical signal of patient 12. In some of examples, electrodes 17A-17C can be platinum coils and/or can be shorted together or electrically isolated to enable independent activation (e.g., delivery or sensing through the respective electrode). In other examples, electrodes 17A-17C can have another electrical connection arrangement.

Loops 54A-54C may be configured to position electrodes 17 in contact (e.g., apposition) with a blood vessel wall or otherwise close to a target tissue site in brain 18 outside of the blood vessel. For example, loops 54A-54C enable endovascular device 50 to make relatively uniform contact along the vessel wall, which may enable a uniform or directional electrical stimulation field in examples where medical device 14 (FIG. 1) delivers electrical stimulation therapy. Medical device 14 may generate the directional electrical stimulation field by selecting (by processing circuitry 30) electrodes 17 that are facing in the desired direction and not delivering electrical stimulation via electrodes 17 that are not facing in the desired direction. The directional electrical stimulation field can help target certain regions of brain 18 while minimizing delivery of electrical stimulation to other regions of brain 18 associated with adverse effects.

FIG. 4D illustrates a two-dimensional end view (e.g., a distal to proximal end view shown as the y-z plane) of expandable element 52 including loop 54A, loop 54B, and loop 54C, wherein each loop defines a flower petal shape (e.g., an obovate flower petal shape, as shown in FIG. 4D). In the example of FIG. 4D, expandable element 52 includes electrode 417A, electrode 417B, electrode 417C, electrode 417C (collectively "electrodes 417"), which may be an example arrangement of electrodes 17 of FIGS. 4A-4C. Electrodes 417 may include all attributes of electrodes 17 described herein. Each of loop 54A, loop 54B, and loop 54C may be circumferentially separated from the respective adjacent loops (e.g., circumferentially separated around elongated body 51, or circumferentially separated around a longitudinal axis 55 of endovascular device 50, as shown in FIG. 4B) . For purposes of illustration, loop 54A is shown with an axis 425, where axis 425 extends radially outward from a center of expandable element 52 (e.g., elongated body 51) and through a point of loop 54A extending furthest away from the center of expandable element 52 (e.g., elongated body 51). Axis 425 can be, for example, aligned with (e.g., intersect) a longitudinal axis of elongated body 51. Similarly, loop 54B is shown with an axis 427, where axis 427 extends radially outward from the center of expandable element 52 (e.g., elongated body 51) and through a point of loop 54B extending furthest away from the center of expandable element 52 (e.g., elongated body 51). Similarly, loop 54C is shown with axis 429, where axis 429 extends radially outward from a center of expandable element 52 (e.g., elongated body 51) and through a point of loop 54C extending furthest away from the center of expandable element 52 (e.g., elongated body 51).

In some examples, loops 54A-54C are spaced apart relative to each other and extend in different directions, which may enable electrodes 417A-417D to face one or more dictions or be placed in apposition with different portions of a blood vessel wall. In the example of FIG. 4D, loop 54A extends in a first direction (e.g., a first direction extending radially outward from a center of expandable element 52 along axis 425, shown as the z direction extending away from elongated body 51). Similarly, loop 54B extends in a second direction (e.g., a second direction extending radially outward from a center expandable element 52 along an axis 427) different than the first direction. Similarly, loop 54C extends in a third direction (e.g., a third direction extending radially outward from a center of expandable element 52 along an axis 429) different than the first direction and the second direction. In this way, loop 54A is disposed at an angle A from loop 54B, wherein angle A is measured between axis 425 of loop 54A and axis 427 of loop 54B. Similarly, loop 54B is disposed at an angle B from loop 54C, wherein angle B is measured between axis 427 of loop 54B and axis 429 of loop 54C. Similarly, loop 54C is disposed at an angle C from loop 54A, wherein angle C is measured between axis 429 of loop 54C and axis 425 of loop 54A.

In some examples, loop 54A, loop 54B, and loop 54C are equally spaced apart (e.g., around elongated body 51, such that angle A, angle B, and angle C are equal). However, in some examples, loop 54A, loop 54B, and loop 54C are not equally spaced apart (e.g., around elongated body 51, such that angle A, angle B, and angle C are not equal). In some examples, the sum of angle A, angle, B, and angle C is 360 degrees.

In some examples, loop 54A, loop 54B, and loop 54C are sized for placing one or more electrodes within the neurovasculature. In some examples, loop 54A, loop 54B, and loop 54C extend radially away from elongated body 51 to place one or more electrodes disposed on each loop in contact with a vessel wall. In the example of FIG. 4D, loop 54A defines a length D1 (measured by a straight line extending between elongated body 51 and a point on a perimeter of loop 54A extending furthest from elongated body 51) and a width D2 (measured by a straight line at the widest point of loop 54A along a line perpendicular to length D1). Length D1 may be measured along points of loop 54A coaxial with axis 425. Similarly, loop 54B defines a length D3 (measured between elongated body 51 and a point on a perimeter of loop 54B extending furthest from elongated body 51) and a width D4 (measured at the widest point of loop 54B along a line perpendicular to length D3).

Length D3 may be measured along points of loop 54B coaxial with axis 427. Similarly, loop 54C defines a length D5 (measured by a straight line between elongated body 51 and a point on a perimeter of loop 54C extending furthest from elongated body 51) and a width D6 (measured by a straight line at the widest point of loop 54C along a line perpendicular to length D5). Length D5 may be measured along points of loop 54C coaxial with axis 429. In some examples, loop 54A, loop 54B, and loop 54C define equal sizes or similar sizes such that length D1, length D3, and length D5 are equal or about equal (e.g., to account for small variations in size, such as to account for manufacturing tolerances) and such that width D2, width D4, and width D5 are equal or about equal (e.g., to account for small variations in size, such as to account for manufacturing tolerances). However, in some examples, loop 54A, loop 54B, and loop 54C do not define equal sizes, such that length D1, length D3, and length D5 are not equal and such that width D2, width D4, and width D5 are not equal. In some examples, one or more loops are the same size, while other loops are not the same size. In some examples, length D1, length D3, and/or length D5 are about 0.5 mm to 4.0 mm, such as 1.50 mm or about 1.50 mm (e.g., account for small variations in size, such as to account for manufacturing tolerances). In some examples, width D2, width D4, and/or width D6 are about 0.5 mm to 4.0 mm, such as 1.50 mm or about 1.50 mm (e.g., account for small variations in size, such as to account for manufacturing tolerances).

FIG. 5A and FIG. 5B illustrate an example endovascular device 60, which is another example of endovascular device 16. FIG. 5A illustrates a first orientation of endovascular device 60, and FIG. 5B illustrates a second orientation of endovascular device 60 where endovascular device 60 is slightly rotated around a longitudinal axis 65 of endovascular device 60 as compared to FIG. 5A. Endovascular device 60 includes a plurality of expandable elements 62A, 62B, 62C longitudinally separated (also referred to herein as longitudinally spaced) from each other along an elongated body 64 along longitudinal axis 65 of endovascular device 60. Longitudinal axis 65 can also correspond to a longitudinal axis of elongated body 64.

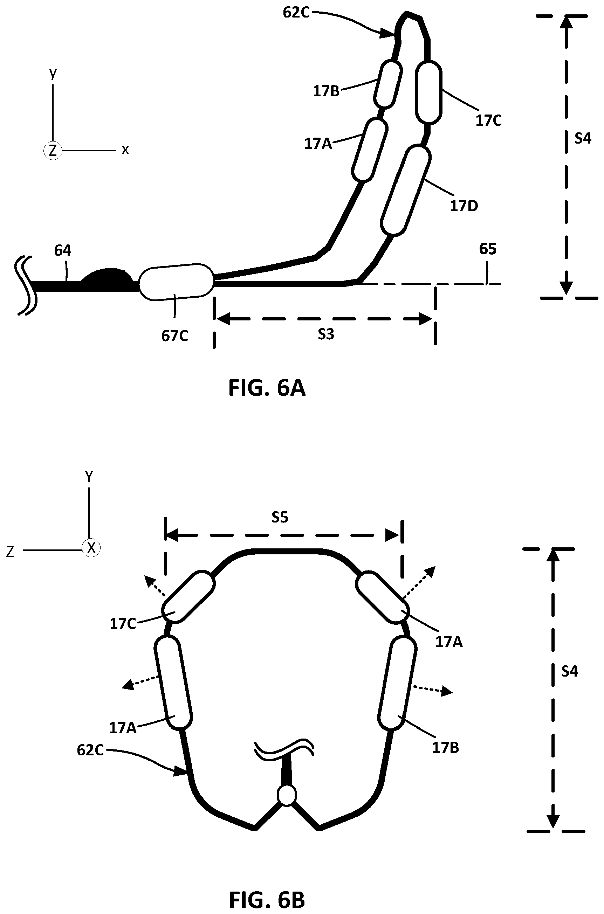

Expandable elements 62A-62C may be examples of expandable element 19 of FIG. 1, and also may be used instead of or in combination with loops 54A-54C of expandable element 52 of FIGS. 4A-4D. Expandable elements 62A-62C are each configured to expand radially outwards from a relatively low-profile delivery configuration (e.g., relatively close to, such as in contact with an outer surface of elongated body 64) to the deployed (e.g., expanded) configuration shown in FIGS. 5A and 5B. FIGS. 6A and 6B illustrates an example close-up view of one of expandable elements 62A-62C, which can have similar configurations or different configurations. For example, expandable elements 62A-62C can have the same size or different sizes, e.g., may increase or decrease in expanded cross-sectional dimension (the cross-section being taken in a direction orthogonal to a longitudinal axis 65 of elongated body 64) in a distal direction or in a proximal direction.

Although three expandable elements 62A-62C are shown in FIGS. 5A and 5B, in other examples, endovascular device 60 can have one expandable element, two expandable elements or more than three expandable elements. Further, although FIGS. 5A and 5B illustrate a single expandable element at a given longitudinal location along elongated body 64, two or more expandable elements similar to expandable elements 62A-62C may be positioned at a given longitudinal location (similar to the example of FIGS. 4A-4D).

Expandable elements 62A-62C can be longitudinally spaced from each other along elongated body 64 and/or can have a common longitudinal position. As shown in the example of FIG. 5A, expandable element 62A is longitudinally spaced from expandable element 62B by a longitudinal distance S1 (e.g., as measured between a proximal-most point of expandable element 62A and proximal-most point of expandable element 62B), and expandable element 62B is longitudinally spaced from expandable element 62C by a longitudinal distance S2 (e.g., as measured between a proximal-most point of expandable element 62B and proximal-most point of expandable element 62C). In some examples, distance S1 and distance S2 are about 3mm-7mm, such as 5mm or about 5mm (e.g., to account for slight variations, such as to account for manufacturing tolerances).

In some examples, expandable elements 62A-62C are equally spaced apart, such that distance S1 is the same or similar to distance S2 (e.g., to account for slight variations, such as to account for manufacturing tolerances). However, in some examples, expandable elements 62A-62C are not equally spaced apart, such that distance S1 is not equal to distance S2. In some examples, a greater separation distance between adjacent expandable elements 62A-62C enables endovascular device 60 deliver electrical stimulation to a larger volume of tissue or enables processing circuitry 30 to better target tissue site with one endovascular device 60 by selecting a subset of electrodes 17 (e.g., one, two or more of electrodes 17, but not all electrodes 17).

As shown in the examples of FIGS. 5A and 5B, one or more electrodes 17 can be positioned on each expandable element 62A-62C, and are configured to deliver electrical stimulation signals (e.g., in a monopolar or bipolar arrangement) and/or sense a patient parameter. In some examples, electrodes 17 define a cylindrical or ring shape. In other examples, electrodes 17 have a partial ring shape. In some examples, electrodes 17 is defined by a laser cut structure (e.g., a laser cut tube). In some examples, the laser cut structure of electrode 17 define one or more finger-like projections. In some examples, expandable elements 62A-62C include the same number of electrodes 17 (e.g., four electrodes, as shown in the examples of FIGS. 5A and 5B). However, expandable elements 62A-62C may each include a different number of electrodes in other examples.

Expandable elements 62A-62C can define any suitable deployed (e.g., expanded) configuration, such as open (e.g., partial) or closed loops (also referred to as hoops or rings) and can be circular, non-circular, or the like. In some examples, expandable elements 62A-62C include one or more crimps 67A-67C. In the example of FIG. 5A and 5B, one or more crimps 67A-67C may join wire portions of expandable elements 62A-62C. For example, crimp 67A joins wires of a proximal portion of expandable element 62A, crimp 67B joins wires of a proximal portion of expandable element 62B, and crimp 67C joins wires of a proximal portion of expandable element 62C. One or more crimps 67A-67C may include radiopaque marker bands. One or more crimps 67A-67C may join one or more loose ends of the wires that form the expandable elements 62A-62C. In some examples, endovascular device 60 includes one or more structural support elements 69. Structural support elements 69 may include radiopaque marker bands. Structural support elements 69 may be configured to join two or more separate wire portions (e.g., conductors) along endovascular device 60.

In some examples, two or more (e.g., all) of the expandable elements 62A-62C are configured to expand in the same direction away from elongated body 64 or can expand in different radial directions, e.g., as shown in FIG. 5A. In some examples, expandable elements 62A-62C extend in the same direction away from elongated body 64 such that at least some electrodes 17 on each respective expandable element of expandable elements 62A-62C are longitudinally aligned (e.g., longitudinally aligned in a direction parallel to longitudinal axis 65, or the "x" direction in the example of FIG. 5A and 5B). As discussed below, in some examples, at least some electrodes 17 on each respective expandable element of expandable elements 62A-62C face the same direction (e.g., a directionally radially outward from each respective expandable element of expandable elements 62A-62C). Some or all of electrodes 17 that face the same direction may be shorted together, regardless of whether expandable elements 62A-62C extend in different radial directions or are aligned.

In some examples, expandable elements 62A-62C are configured to position electrodes 17 in contact with a blood vessel wall or otherwise close to a target tissue site in brain 18 outside of the blood vessel. For example, expandable elements 62A-62C can enable endovascular devices 60 to make relatively uniform contact along the vessel wall and deliver electrical stimulation to provide a uniform or directional electrical stimulation field. Medical device 14 can generate the directional electrical stimulation field by selecting (by processing circuitry 30) electrodes 17 that are facing in the desired direction and not delivering electrical stimulation via electrodes 17 that are not facing in the desired direction. The directional electrical stimulation field can help target certain regions of brain 18 while minimizing delivery of electrical stimulation to other regions of brain 18 associated with adverse effects.

In some examples, expandable elements 62A-62C can include electrodes 17 positioned and arranged to provide a horizonal directional field, e.g., along one side of longitudinal axis 65 or otherwise along less than 360 degrees around longitudinal axis 65.

FIGS. 6A and 6B illustrate an example arrangement of electrodes 17 on expandable element 62C from FIGS. 5A and 5B. In particular, FIGS. 6A and 6B illustrates an example expandable element 62C (which can be representative of the configuration of expandable elements 62A and 62B in some examples), which defines a closed loop and includes four electrodes 17A, 17B, 17C, and 17D. In some examples, electrodes 17A-17D are independently activatable to enable processing circuitry 30 to select the one or more electrodes of electrodes 17A-17D with which electrical stimulation can be delivered to generate the desired electric field or activation field (e.g., in a particular direction away from longitudinal axis 65, rather than 360 degrees around longitudinal axis 65). In other examples, two or more of electrodes 17A-17D can be shorted together. For example, electrode 17A and electrode 17B can be shorted together, and electrode 17C and electrode 17D can be separately shorted together (e.g., separate from electrode 17A and electrode 17B). In some examples, each of electrodes 17A-17D face in different directions (e.g., different directions extending radially outward from the "x" axis in the example of FIG. 6B) relative to the other electrodes of electrodes 17A-17D on expandable element 62C.

Expandable element 62C may be sized to be introduced into one or more vessels within the neurovasculature and deployed to position one or more electrodes (e.g., electrodes 17A-17D) into apposition with a blood vessel wall. In some examples, when deployed, expandable element 62C defines a longitudinal distance S3 between a proximal-most point of expandable element 62C and a distalmost point of expandable element 62C (as measured along a longitudinal axis, such as parallel to longitudinal axis 65). In some examples, longitudinal distance S3 is about 2mm-4mm, such as 3mm or about 3mm (e.g., to account for slight variations, such as to account for manufacturing tolerances). In some examples, when deployed, expandable element 62C defines a radial distance S4 between a point on expandable element 62C closest to elongated body 64 and a point extending furthest away from elongated body 64 (e.g., as measured perpendicular to longitudinal axis 65). In some examples, radial distance S4 is about 2mm-4mm, such as 3mm or about 3mm (e.g., to account for slight variations, such as to account for manufacturing tolerances). In some examples, when deployed, expandable element 62C defines a widest internal dimension S5 (e.g., diameter, in examples where expandable element includes a circular or oval shape). In some examples, widest internal dimension S5 is about 1mm-3mm, such as 2mm or about 2mm (e.g., to account for slight variations, such as to account for manufacturing tolerances).

Loops 54A-54C or expandable elements 62A-62C are formed from any suitable material, such as a shape memory material (e.g., nitinol). For example, each loop 54A-54C or expandable element 62A-62C can be formed from a nitinol wire, e.g., about 0.05 mm to about 0.1 mm in diameter, such as 0.075 mm or about 0.075 mm (e.g., to account for slight variations, such as to account for manufacturing tolerances), which is approximately about 0.076mm (0.003 inches) in diameter.

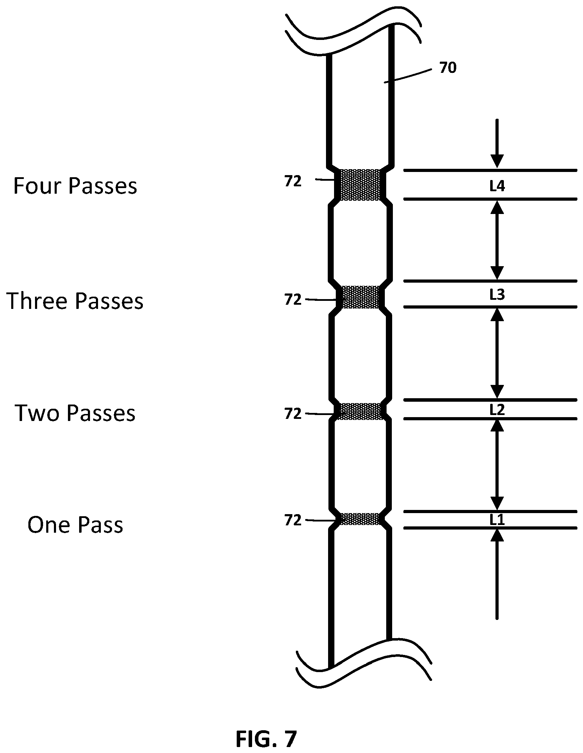

In some examples, some or all of the electrodes 17 of the respective endovascular device 50, 60 are integrally formed with loops 54A-54C or expandable elements 62A-62C. For example, loops 54A-54C and/or expandable elements 62A-62C can be formed from an electrically conductive material (e.g., a nitinol core wire) that is electrically connected to therapy generation circuitry 34 and sensing circuitry 36 (FIG. 3) and an electrically insulative material can be positioned radially outwards of the electrically conductive material, e.g., to cover the electrically conductive material. To define electrodes 17, part of the electrically insulative material can be removed (e.g., via laser ablation, mechanical etching, or the like) to expose the electrically conductive material, e.g., as shown with respect to FIG. 7. Any suitable electrically insulative material can be used, such as, but not limited to, polytetrafluoroethylene (PTFE), expanded PTFE (ePTFE), silicone, polyimide, non-metallic oxide, or the like. The electrically insulative material can have any suitable thickness, such as, but not limited to, 0.010 mm to 0.05 mm (e.g., about 0.0127mm (0.0005 inches)).

In other examples, some or all of the electrodes 17 of the respective endovascular device 50, 60 are or include a component physically separate from loops 54A-54C or expandable elements 62A-62C and mechanically connected to the respective loops 54A-54C or expandable elements 62A-62C. For example, an electrically conductive electrode material (e.g., platinum, tungsten, gold, or the like, which can be radiopaque or not) can be electrically connected to the electrically conductive material core material discussed above, which can function as an electrical conductor. The electrode material can be in any suitable form, such as a coil that is wrapped around the electrically conductive material core material, a marker band, a material (e.g., gold or platinum) plated over the electrically conductive core, or the like.

In some examples, an expandable element of an endovascular device, such as each of the loops 54A-54C or expandable elements 62A-62C, be formed from several wires bundled to facilitate shaping into the desired form (e.g., circular shape).

An expandable element of an endovascular device, such as each of the loops 54A-54C or expandable elements 62A-62C, can include any suitable number of electrodes 17, such as one, two, three or more electrodes. Some or all of the electrodes 17 of the particular endovascular device 16 can be separately electrically connected to medical device 14 to enable processing circuitry 30 to selectively and independently activate one or more electrodes 17 (while not activating one or more of the other electrodes 17 on the same expandable element or a different expandable element).

In addition to some or all of the electrodes 17 being separately (also referred to herein as independently) activatable, some or all of the electrodes 17 (e.g., on the same loop or expandable element or different loops or expandable elements) can be electrically connected together (also referred to as shorted together in some examples). In some examples, shorting some or all of electrodes reduces the overall profile of endovascular devices 16, 50, 60 by reducing the number of electrical conductors required to electrically connect electrodes 17 to medical device 14.

Each of the aforementioned endovascular devices 16, 50, and 60 may include electrode arrangements or other features that facilitate directional stimulation and/or directional sensing, which may also reduce the number of conductors that need to be connected to the electrodes. In some examples, electrodes facing in the same direction can be shorted together. Although shorting electrodes together may not enable processing circuitry 30 to activate each electrode 17 of endovascular device 60 individually, shorting electrodes together may enable directional delivery of electric fields through various combinations of electrodes 17 and direction sensing through various combinations of electrodes 17. Multiple electrodes 17 on a particular expandable element or on multiple expandable elements and facing in different directions (e.g., different radial directions) can help enable directional delivery of electric field to help achieve targeted therapy delivery to brain 18.

In some examples, electrodes on different loops of a given expandable structure that face in the same direction are shorted together. In some examples, with reference to FIG. 4D, electrodes that face in the same direction on different loops (e.g., loop 54A and 54B) are shorted together. In some examples, a first loop or expandable element includes a first electrode facing in a first direction (e.g., as shown in the example of FIG. 4D, loop 54A includes electrode 417A facing in the z-axis direction) and a second electrode facing in a second direction different from the first direction (e.g., as shown in the example of FIG. 4D, loop 54A includes electrode 417B facing in the y-axis direction) and a second loop or expandable element includes a third electrode facing in the first direction (e.g., as shown in the example of FIG. 4D, loop 54B includes electrode 417C facing in the z-axis direction) and a fourth electrode facing in the second electrode (e.g., as shown in the example of FIG. 4D, loop 54B includes electrode 417D facing in the y-axis direction). In this example, the first and third electrodes are shorted together (e.g., in the example of FIG. 4D, electrode 417A and 417C can be shorted together) and the second and fourth electrodes are shorted together (e.g., in the example of FIG. 4D, electrode 417B and 417D can be shorted together).

In some examples, electrodes that face the same direction on different expandable elements are shorted together. For example, with reference to endovascular device 60 of FIGS. 5A, 5B, 6A, and 6B, each expandable element 62A-62C includes four electrodes (e.g., electrode 17A, electrode 17B, electrode 17C, and electrode 17D as shown in FIG. 6B) that face in different directions (e.g., different directions extending radially outward from the x axis in the example of FIG. 6B) relative to electrodes on a given expandable element, but that may face the same direction as corresponding electrodes on a different expandable element. In some examples, electrodes 17 of expandable elements 62A-62C facing in the same respective direction are shorted together, such that endovascular device 60 has four electrical conductors for twelve electrodes. In some examples, each expandable element 62A-62C includes three electrodes that face in different directions relative to electrodes on a given expandable element but face the same direction as corresponding electrodes on a different expandable element. In some examples, the electrodes 17 of the expandable elements 62A-62C facing in the same respective direction are shorted together, such that endovascular device 60 has three electrical conductors for nine electrodes. Any number of conductors and electrodes may be used (e.g., three conductors for six electrodes, etc.).

The endovascular devices described herein, including endovascular devices 16, 50, 60 can be delivered using any suitable device, which can be selected based on the intracranial vessel of interest. As an example, the endovascular devices described herein can be delivered to an intracranial blood vessel inside of a 0.43 mm (e.g., about 0.017-inch) or a 0.53 mm (e.g., about 0.021-inch) microcatheter, alone or with the aid of a guidewire.

FIG. 7 illustrates an example of how electrodes 17 may be defined by selectively removing an electrically insulative material 70 (e.g., via laser ablation, mechanical etching, or the like) to expose an electrically conductive material 72 radially inward of the electrically insulative material 70. The exposed electrically conductive material 72 can define electrodes 17 or can be used to connect another electrically conductive material (e.g., an electrode coil or an electrode band) to conductive material 72. FIG. 7 illustrates, for example, how multiple passes can be made in order to increase the length (measured in a longitudinal direction of the endovascular device) of the exposed electrically conductive material. For example, a single pass may be associated with a width (e.g., L1) of material removed (e.g., via laser ablation, mechanical etching, or the like), such that multiple successive passes adjacent to each other increases the width of the exposed conductive material 72. Exposed length of electrically conductive material 72 increases from L1 to L2 to L3 to L4 with each pass of the material removal (e.g., laser ablation), which can be limited by the size of the cutting device or laser. Although four passes are shown in FIG. 7, any suitable number of passes, including one, two, three, or more than four, can be used to define electrodes 17.

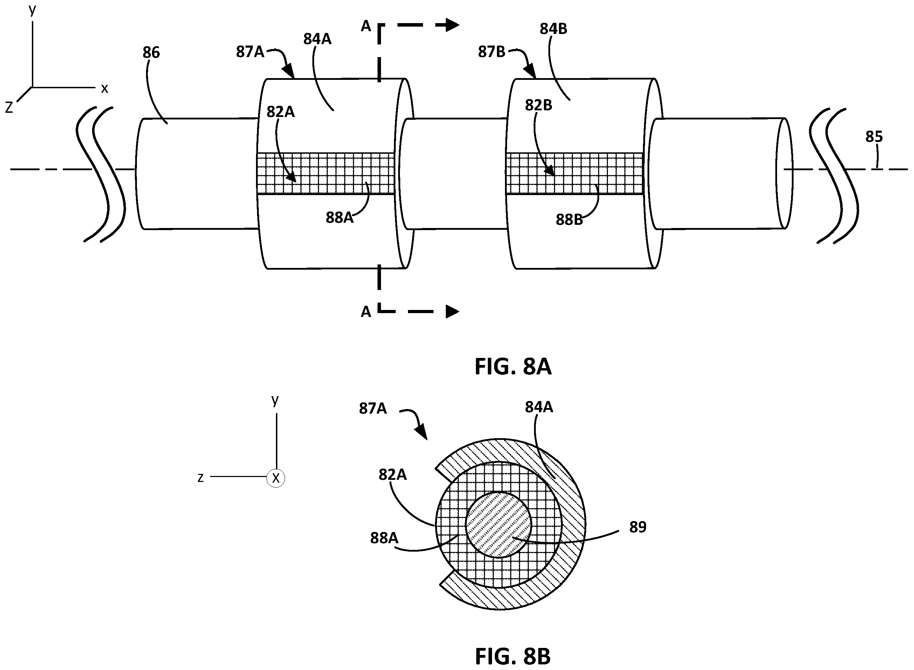

FIGS. 8A and 8B illustrate an example of how electrodes may be partially covered by electrically insulative material to facilitate directional stimulation and/or directional sensing. In the example perspective view of FIG. 8A, an elongated body 86 (which may be an example of elongated body 51, elongated body 64, or another suitable electrode carrying portion), includes electrodes 87A and 87B (collectively "electrodes 87", which may be examples of electrodes 17) spaced longitudinally along elongated body 86. Electrodes 87 may be spaced longitudinally along elongated body 86 along a central longitudinal axis 85 of elongated body 86 (extending in the x-axis direction in the example of FIGS. 8A and 8B, where orthogonal x-y-z axes are shown in FIGS. 8A and 8B for ease of description).

Electrode 87A includes electrically insulative material 84A surrounding at least a portion of an outer perimeter (e.g., outer diameter in examples in which electrode 87A has a circular cross-section) of an electrically conductive material 88A. Similarly, electrode 87B includes electrically insulative material 84B surrounding at least a portion of an outer perimeter (e.g., outer diameter in examples in which electrode 87B has a circular cross-section) of an electrically conductive material 88B. Insulative material 84A and insulative material 84B (collectively "insulative material 84") may comprise a suitable electrically insulative material (e.g., a polymer), such that when insulative material 84 is positioned between conductive material 88A and conductive material 88B (collectively "conductive material 88") and tissue of a patient, insulative material 84 reduces or even prevents conductive material 88 from transmitting electrical signals to and/or sensing signals from tissue of a patient.

In some examples, electrodes 87 include conductive material 88 at least partially covered by insulative material 84 such that only a section 82A and a section 82B (collectively "sections 82") of conductive material 88 is exposed (e.g., exposed to blood and/or tissue within a blood vessel of patient 12). Sections 82 of exposed conductive material 88 may face in a direction (e.g., a direction radially outward from central longitudinal axis 85) to facilitate directional stimulation and/or directional sensing. Additionally, using sections 82 of exposed conductive material 88 on elongated body 86 may have an overall lower profile as compared to other alternatives. In the example of FIG. 8A, section 82 generally faces in the z-axis direction, which is radially outward from longitudinal axis 85 of elongated body 86. In use, elongated body 86 may be rotated to position section 82 adjacent a target location within the vasculature for stimulation and/or sensing via electrodes 87.

Sections 82 may be formed using any suitable method. In some examples, a method of forming includes covering conductive material 88 (e.g., an entire outer surface of conductive material 88) with electrically insulative material 84, and subsequently removing (e.g., ablating) portions of the electrically insulative material 84 to define sections 82. In some examples, electrically insulative material 84 is added to conductive material 88 such that when electrically insulative material 84 is added, a gap or opening in insulative material 84 defines sections 82.

Electrodes 87 may include a suitable number and orientations of exposed sections 82 of conductive material 88 (e.g., depending on the type of expandable element used, the target tissue site within brain 18, and the like). In some examples, each electrode of electrodes 87 includes more than one section 82 of exposed conductive material. In some examples, sections 82 of exposed conductive material 88 on elongated body 86 may act as segmented electrodes (in examples with multiple sections 82). In some examples, electrodes 87 include multiple sections 82 rotationally spaced apart along the circumference from each respective electrode of electrodes 87 (e.g., where each of electrode 87A and 87B include a first exposed section 82 generally facing the z-axis direction as shown in FIG. 8A, and another exposed section generally facing the y-axis direction). In some examples, exposed section 82A of electrode 87A and exposed section 82B of electrode 87B face in the same direction (e.g., the z-axis direction, as shown in the example of FIG. 8A). However, in some examples, exposed section 82A of electrode 87A and exposed section 82B of electrode 87B face in different directions (e.g., exposed section 82A faces the z-axis direction, and exposed section 82B faces in the y-axis direction).

FIG. 8B illustrates a cross section of an example elongated body and electrodes of FIG. 8A, the cross-section taken through a plane transverse (e.g., orthogonal) to the longitudinal axis 85 of elongated body 86 (e.g., section A-A indicated in FIG. 8A). As discussed in connection with FIG. 8A, electrode 87A includes insulative material 84A around an outer perimeter of electrode 87A. For example, insulative material 84A may cover (e.g., be radially outwards of) at least a portion of a conductive material 88A to define at least one exposed section 82A of conductive material 88A is exposed. In some examples, electrode 87A includes a core 89 radially inward from conductive material 88A and insulative material 84A. Core 89 may include another conductive material (e.g., the same material as conductive material 88), or more may include one or more wires (e.g., coated wires) that act as conductors for electrodes 87 e.g., to connect electrodes 87 to a medical device including electrical stimulation generation circuitry and/or sensing circuitry.

Although FIGS. 8A and 8B illustrate electrodes 87 as protruding (e.g., in the y-axis and z-axis directions) from an outermost surface of elongated body 86, in some examples, electrodes 87 are formed to be flush or about flush with the outermost surface of elongated body 86.

As discussed above, the devices of this disclosure may be configured to generate one or more electrical stimulation waveforms. In some examples, the electrical stimulation waveform generated by medical device 14 and which may be delivered by one or more of electrodes 17 is a charge balanced, biphasic waveform. In some examples, such an electrical stimulation waveform consist of periodic pulses or otherwise include periodic pulses, or can include a continuous time waveform.

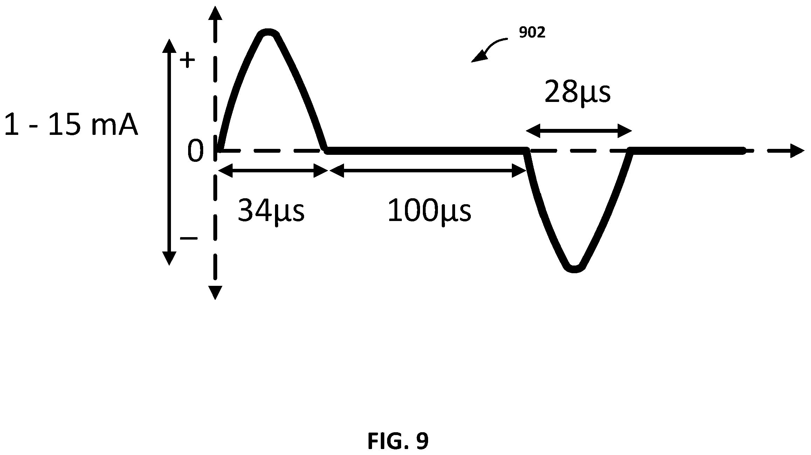

FIG. 9 is a conceptual diagram illustrating an example electrical stimulation waveform that can be generated by therapy generation circuitry 34 of medical device 14 (FIG. 3) and which may be delivered by one or more of electrodes 17 according to one or more aspects of this disclosure. Waveform 902 is an example of an electrical stimulation waveform which one or more of electrodes 17 may deliver endovascularly. Waveform 902 is an example of a charge balanced waveform. In some examples, waveform 902 includes a first period of stimulation of 34 microseconds (µs), a second period of stimulation of 28 microseconds (µs), and a period of no stimulation of 100 microseconds (µs) between the first period of stimulation and the second period of stimulation. In some examples, first stimulation period includes a positive charge and the second stimulation period includes a negative charge.

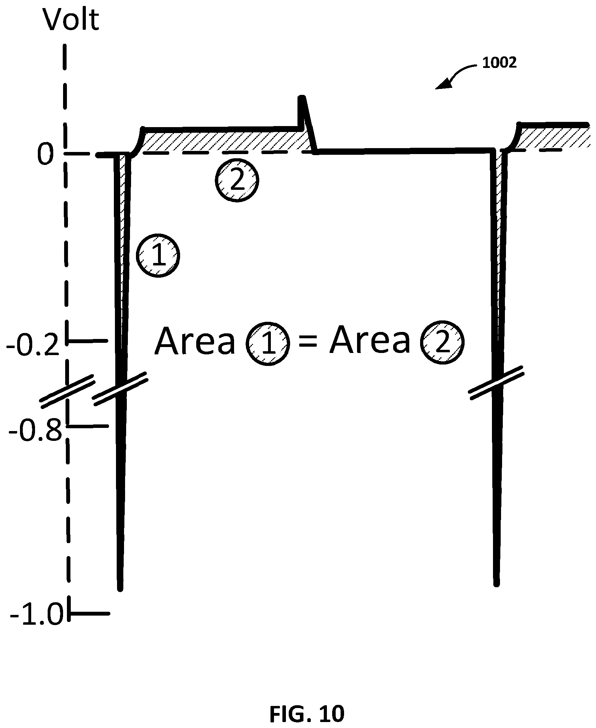

FIG. 10 is a conceptual diagram illustrating another example electrical stimulation waveform generated by therapy generation circuitry 34 of medical device 14 and which may be delivered by one or more of electrodes 17 according to one or more aspects of this disclosure. Waveform 1002 is an example of an electrical stimulation waveform which one or more of electrodes 17 may deliver endovascularly. Waveform 1002 is another example of a charge balanced waveform. In some examples, waveform 1002 includes a first portion with a first area under the curve of the waveform (indicated by "area 1") and a second portion with a second area under the curve under the waveform (indicated by "area 2"), where the first area is equal to the second area.

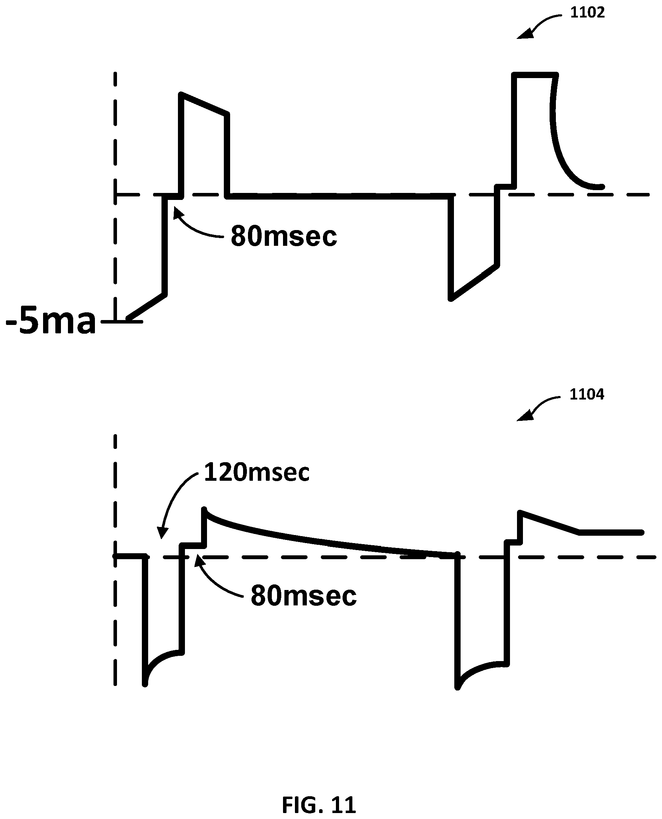

FIG. 11 is a conceptual diagram illustrating further example electrical stimulation waveforms generated by therapy generation circuitry 34 of medical device 14 and which may be delivered by one or more of electrodes 17 according to one or more aspects of this disclosure. Both waveform 1102 and waveform 1104 are examples of electrical stimulation waveforms which one or more of electrodes 17 may deliver endovascularly. Waveform 1102 may be an example of a charge balanced waveform.

Processing circuitry 30 (FIG. 3) may control therapy generation circuitry 34 to generate the electrical stimulation waveform to be delivered by one or more of electrodes 17. The electrical stimulation waveform may reflect one or more therapy parameters. In some examples, the therapy parameters may include an amplitude ranging between 0.01 milliamps (mA) and 25 mA, inclusive, a time period ranging from 0.1 microseconds (µs) and 5 minutes, inclusive, a frequency ranging from 1 Hertz (Hz) to 1 megahertz (MHz), inclusive, or any combination thereof. For example, any of waveforms 902, 1002, 1102, or 1104 may include such therapy parameters.

Endovascular devices (including but not limited to leads, catheters, stents, and the like), such as endovascular device 16, may be placed (e.g., inserted and/or guided) in venous or arterial vessels closest to the target tissue. Such target tissue may include, but not be limited to, Anterior nucleus of the thalamus (ANT), hippocampus (HIP), subthalamic nucleus (STN), the Fornix, the internal segment and/or external segments of the globus pallidus, or the ventral intermediate nucleus of the thalamus. The vasculature into which endovascular device 16 may be inserted and/or guided includes, but is not limited to, the Thalamostriate vein, internal cerebral vein, basal vein of Rosenthal, inferior/superior sagittal sinus, or arterial vessels including, but not limited to, anterior choroidal artery or any related combinations thereof.

Certain vessels into which endovascular device 16 may be inserted and/or guided may be located at different distances from different target tissue. Such distances may play a role in efficacy of therapy delivered by endovascular device 16, as a closer distance may indicate a shorter distance any electrical stimulation waveform may have to travel, and, in some examples, the less power that is needed to generate the electrical stimulation waveform. Also, different vessels of the vasculature may have different diameters which may affect a design of endovascular device 16 to permit endovascular device 16 entry into such vasculature. For example, if a diameter of endovascular device 16 is not smaller than the diameter of the vessel, endovascular device 16 may not be introduced into that vessel.

For example, the Thalamostriate vein may be approximately 1.2 mm in diameter and be located 0-2 mm from the ANT and approximately 0-2 mm from the Fornix. The internal cerebral vein may be 1.9 mm plus or minus up to 0.5 mm in diameter and be located approximately 5-10 mm from the ANT and 2-5 mm from the Fornix. The basal vein of Rosenthal may be 1.7 mm plus or minus up to 0.4 mm in diameter and be located approximately 10-15 mm from the ANT, approximately 5-10 mm from the HIP, and approximately 5-10 mm from the STN. The inferior sagittal sinus may be 1.3 mm plus or minus up to 0.3 mm in diameter and be located 10-15 mm from the Fornix. Thus, selection of an appropriate vessel from which to stimulate the target tissue may be desirable.

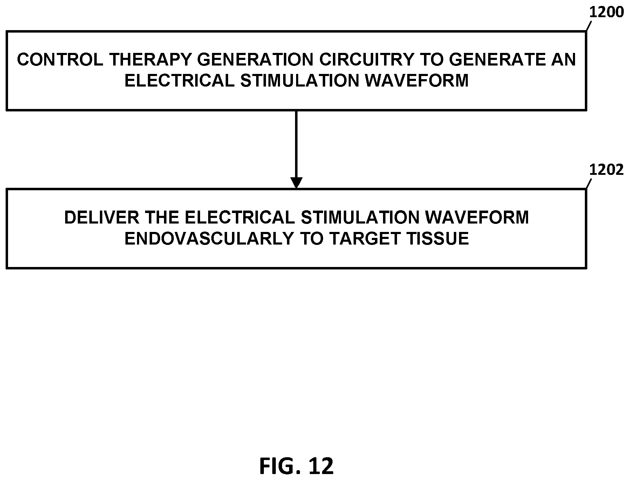

FIG. 12 is a flow diagram illustrating example techniques for delivering endovascular deep brain stimulation according to one or more aspects of this disclosure. Processing circuitry 30 controls therapy generation circuitry 34 to generate an electrical stimulation waveform (1200). For example, processing circuitry 30 may control therapy generation circuitry 34 to generate an electrical stimulation waveform including a charged balanced and biphasic waveform. One or more electrodes of electrodes 17 may deliver the electrical stimulation waveform endovascularly to target tissue (1202). For example, the one or more of electrodes 17 may be electrically coupled to therapy generation circuitry 34 and may deliver the electrical stimulation waveform to the target tissue.

In some examples, endovascular device 16 is configured to be inserted at least partially into a vasculature of a patient. In some examples, each of the one or more electrodes of electrodes 17 is disposed on endovascular device 16 at a respective location. In some examples, the electrical stimulation waveform is to treat a patient condition.

In some examples, the respective location includes a distal portion of the device, a central portion of the device, or a proximal portion of the device. In some examples, the respective location comprises a circumferential location at any location along a length of the device. In some examples, the respective location comprises a radiopaque marker.

In some examples, at least one of the one or more electrodes of electrodes 17 is configured to deliver directional stimulation and/or directional sensing. In some examples, a number of electrodes of electrodes 17 is in a range of 1 electrode to 50 electrodes, inclusive. In some examples, endovascular device 16 includes one or more leads and the one or more electrodes of electrodes 17 are disposed on the one or more leads. In some examples, a number of the one or more leads is in a range of 1 lead to 50 leads.

In some examples, the one or more electrodes of electrodes 17 have a respective shape, the respective shape including square, rectangular, circular, spherical, oval, cubic, ovoid, or cuboid. In some examples, the one or more electrodes of electrodes 17 have a respective surface area, wherein the respective surface area is in a range of 0.01 square millimeters to 200 square millimeters, inclusive.

In some examples, the vasculature includes at least one of a Thalamostriate vein, an internal cerebral vein, a basal vein of Rosenthal, an inferior sagittal sinus, a superior sagittal sinus, or an arterial vessel. In some examples, the arterial vessel includes an anterior choroidal artery.

In some examples, the target tissue includes at least one of an anterior nucleus of thalamus, a hippocampus, a subthalamic nucleus, or a Fornix.

In some examples, the electrical stimulation waveform includes therapy parameters. In some examples, the therapy parameters include at least one of: an amplitude in a range between 0.01 mA to 25 mA, inclusive; a time period in a range between 0.1 microseconds (µsec) to 5 minutes, inclusive; or a frequency in a range between 1 Hz to 1 MHz, inclusive.

In some examples, a method of using therapy system 10 comprises introducing endovascular device 16 into vasculature (e.g., an intracranial blood vessel) of a patient via an access point (e.g., a femoral artery or a radial artery). In some examples, the method includes pushing an elongated body (e.g., elongated body 51 of FIG. 4A or elongated body 64 of FIG. 5A) through the vasculature until electrodes 17 of endovascular device 16 are positioned proximate a target tissue site in brain 18. In some examples, endovascular device 16 is guided to the target tissue site through an inner lumen of an outer sheath or other outer catheter (e.g., as discussed with respect to endovascular device 50 in FIG. 4A, which includes outer sheath 53). The outer sheath or other outer catheter keeps expandable element 19 of endovascular device 16 in a low-profile delivery configuration to help facilitate navigation of endovascular device 16 through vasculature to brain 18. In some examples, such as the examples described above, a separate guidewire is not used with endovascular device 16. However, in some examples, a separate guidewire or inner catheter is first introduced into the vasculature to facilitate navigation of endovascular device 16 to the target tissue site. For example, a guidewire and/or inner catheter may first be introduced into the vasculature, and endovascular device 16 may be delivered over the guidewire and/or the inner catheter.

Once electrodes 17 of endovascular device 16 are placed proximate a target tissue site in brain 18, expandable element 19 is deployed from a low-profile configuration to a deployed configuration. Deploying expandable element 19 may include actuating a pullwire, applying a stimulus to endovascular device 16 (e.g., an energy stimulus to cause expandable element 19 to expand), advancing endovascular device 16 distally of a sheath or other outer catheter (e.g., outer sheath 53, as discussed in connection with FIG. 4A), withdrawing the sheath or other outer member proximally with respect to endovascular device 16, or any combination thereof. In some examples, the method further includes identifying or confirming a position of endovascular device 16, including one or more electrodes 17 (e.g., in relation to a target location within patient 12). The position of endovascular device 16, including one or more electrodes 17, may be identified or confirmed before and/or after deployment of expandable element 19. The position of endovascular device 16, including the position of one or more electrodes 17, may be identified or confirmed via one or more imaging techniques, including x-ray, fluoroscopy, computerized tomography (CT), magnetic resonance imaging (MRI), or another suitable method. In some examples, the method further includes repositioning endovascular device 16, including electrodes 17, within the vasculature of patient 12 (e.g., by retracting endovascular device 16 back into a sheath or other outer catheter to transform endovascular device 16 back to the low-profile configuration). Repositioning of endovascular device 16, including electrodes 17, may be based on, at least in part, imaging data, feedback from a trial period of stimulation and/or sensing, patient feedback, or other feedback.

The techniques described in this disclosure, including those attributed to medical device 14, programmer 20, or various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as clinician or patient programmers, medical devices, or other devices. Processing circuitry, control circuitry, and sensing circuitry, as well as other processors and controllers described herein, may be implemented at least in part as, or include, one or more executable applications, application modules, libraries, classes, methods, objects, routines, subroutines, firmware, and/or embedded code, for example. In addition, analog circuits, components and circuit elements may be employed to construct one, some or all of the processing circuitry 30, instead of or in addition to the partially or wholly digital hardware and/or software described herein. Accordingly, analog or digital hardware may be employed, or a combination of the two.

In one or more examples, the functions described in this disclosure may be implemented in hardware, software, firmware, or any combination thereof. If implemented in software, the functions may be stored on, as one or more instructions or code, a computer-readable medium and executed by a hardware-based processing unit. The computer-readable medium may be an article of manufacture including a non-transitory computer-readable storage medium encoded with instructions. Instructions embedded or encoded in an article of manufacture including a non-transitory computer-readable storage medium encoded, may cause one or more programmable processors, or other processors, to implement one or more of the techniques described herein, such as when instructions included or encoded in the non-transitory computer-readable storage medium are executed by the one or more processors. Example non-transitory computer-readable storage media may include RAM, ROM, programmable ROM (PROM), erasable programmable ROM (EPROM), electronically erasable programmable ROM (EEPROM), flash memory, a hard disk, a compact disc ROM (CD-ROM), a floppy disk, a cassette, magnetic media, optical media, or any other computer readable storage devices or tangible computer readable media.

In some examples, a computer-readable storage medium comprises non-transitory medium. The term "non-transitory" may indicate that the storage medium is not embodied in a carrier wave or a propagated signal. In certain examples, a non-transitory storage medium stores data that can, over time, change (*e.g.,* in RAM or cache).

The functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements.

In some examples, the patient condition includes at least one of epilepsy, Parkinson's disease, dystonia, cerebellar outflow tremor, major depressive disorder (MDD), bipolar disorder, anxiety disorders, post-traumatic stress disorder, dysthymic disorder, or obsessive-compulsive disorder.

Various examples of the disclosure have been described. Any combination of the described systems, operations, or functions is contemplated.

## Claims

1. An endovascular medical device (50) comprising:
an elongated body (51) configured to be introduced in a cranial blood vessel of a patient; and
an expandable element (52) on the elongated body,
wherein the expandable element includes a plurality of loops (54A, 54B, 54C),
wherein each loop includes one or more electrodes (17A, 17B, 17C), and
wherein the expandable element is configured to expand radially outwards from a relatively low-profile delivery configuration to a deployed configuration to position the one or more electrodes to deliver electrical stimulation to tissue of a brain of a patient or sense a patient parameter from a location within the cranial blood vessel, **characterised in that** each loop defines a flower petal shape, and wherein the plurality of loops is distributed around an outer perimeter of the elongated body.

2. The endovascular medical device (50) of claims 1, wherein at least two electrodes (17A, 17B) of at least one loop (54A) of the plurality of loops (54A, 54B, 54C) are independently activatable.

3. The endovascular medical device (50) of any of claims 1 or 2, wherein at least two electrodes (17A, 17B) of at least one loop (54A) of the plurality of loops (54A, 54B, 54C) are shorted together.

4. The endovascular medical device (50) of any of claims 1 through 3, wherein each loop of the plurality of loops (54A, 54B, 54C), includes a plurality of electrodes (17A, 17B, 17C), and wherein electrodes of the loops that face in a same direction are shorted together.

5. The endovascular medical device (60) of any of claims 1 through 4, wherein the expandable element (62) includes a plurality of expandable elements (62A, 62B, 62C), each expandable element including one or more electrodes (17).

6. The endovascular medical device (60) of claim 5, wherein each expandable element (62A, 62B, 62C) of the plurality of expandable elements (62) is longitudinally separated from adjacent expandable elements along a longitudinal axis of the elongated body (64).

7. The endovascular medical device (60) of any of claims 5 or 6, wherein at least two electrodes (17) of at least one expandable element (62A, 62B, 62C) of the plurality of expandable elements (62) are independently activatable.

8. The endovascular medical device (60) of any of claims 5 through 7, wherein at least two electrodes (17) of at least one expandable element of the plurality of expandable elements (62A, 62B, 62C) are shorted together.

9. The endovascular medical device (60) of any of claims 5 through 8, wherein each expandable element of the plurality of expandable elements (62A, 62B, 62C) includes a plurality of electrodes (17), and wherein electrodes of the expandable elements that face in a same direction are shorted together.

10. The endovascular medical device (50, 60) of any of claims 1 through 9, wherein the expandable element (52, 62) includes a shape memory material.

11. The endovascular medical device (50, 60) of any of claims 1 through 10, wherein the expandable element (52, 62) includes an electrically conductive material radially inwards of an electrically insulative material, wherein the at least a portion of the electrically insulative material is removed to expose the electrically conductive material.

12. The endovascular medical device (50, 60) of claim 11, wherein the exposed electrically conductive material defines at least one electrode (17) of the one or more electrodes.

13. The endovascular medical device (50, 60) of any of claims 11 or 12, further comprising an electrode material connected to the expandable element (52, 62) and in electrical contact with the exposed electrically conductive material.

14. The endovascular medical device (50, 60) of claim 13, wherein the electrode material includes one or more of a coil or a band.

## Patentansprüche

1. Endovaskuläre medizinische Vorrichtung (50), umfassend:
ein länglicher Körper (51), der konfiguriert ist, um in ein kraniales Blutgefäß eines Patienten eingeführt zu werden; und
ein dehnbares Element (52) auf dem länglichen Körper,
wobei das dehnbare Element eine Vielzahl von Schleifen (54A, 54B, 54C) einschließt,
wobei jede Schleife eine oder mehrere Elektroden (17A, 17B, 17C) einschließt, und
wobei das dehnbare Element konfiguriert ist, um sich von einer relativ flachen Abgabekonfiguration radial nach außen in eine erweiterte Konfiguration auszudehnen, um die eine oder mehrere Elektroden zu positionieren, um eine elektrische Stimulation an das Gewebe eines Gehirns eines Patienten abzugeben oder einen Patientenparameter von einem Ort innerhalb des kranialen Blutgefäßes zu erfassen, **dadurch gekennzeichnet, dass** jede Schleife eine Blütenblattform definiert, und wobei die Vielzahl von Schleifen um einen äußeren Umfang des länglichen Körpers verteilt ist.

2. Endovaskuläre medizinische Vorrichtung (50) nach Anspruch 1, wobei mindestens zwei Elektroden (17A, 17B) mindestens einer Schleife (54A) der Vielzahl von Schleifen (54A, 54B, 54C) unabhängig voneinander aktivierbar sind.

3. Endovaskuläre medizinische Vorrichtung (50) nach einem der Ansprüche 1 oder 2, wobei mindestens zwei Elektroden (17A, 17B) mindestens einer Schleife (54A) der Vielzahl von Schleifen (54A, 54B, 54C) miteinander kurzgeschlossen sind.

4. Endovaskuläre medizinische Vorrichtung (50) nach einem der Ansprüche 1 bis 3, wobei jede Schleife der Vielzahl von Schleifen (54A, 54B, 54C) eine Vielzahl von Elektroden (17A, 17B, 17C) einschließt und wobei Elektroden der Schleifen, die in die gleiche Richtung weisen, miteinander kurzgeschlossen sind.

5. Endovaskuläre medizinische Vorrichtung (60) nach einem der Ansprüche 1 bis 4, wobei das dehnbare Element (62) eine Vielzahl von dehnbaren Elementen (62A, 62B, 62C) einschließt, wobei jedes dehnbare Element eine oder mehrere Elektroden (17) einschließt.

6. Endovaskuläre medizinische Vorrichtung (60) nach Anspruch 5, wobei jedes dehnbare Element (62A, 62B, 62C) aus der Vielzahl der dehnbaren Elemente (62) in Längsrichtung von benachbarten dehnbaren Elementen entlang einer Längsachse des länglichen Körpers (64) getrennt ist.

7. Endovaskuläre medizinische Vorrichtung (60) nach einem der Ansprüche 5 oder 6, wobei mindestens zwei Elektroden (17) mindestens eines dehnbaren Elements (62A, 62B, 62C) aus der Vielzahl der dehnbaren Elemente (62) unabhängig voneinander aktivierbar sind.

8. Endovaskuläre medizinische Vorrichtung (60) nach einem der Ansprüche 5 bis 7, wobei mindestens zwei Elektroden (17) mindestens eines dehnbaren Elements aus der Vielzahl der dehnbaren Elemente (62A, 62B, 62C) miteinander kurzgeschlossen sind.

9. Endovaskuläre medizinische Vorrichtung (60) nach einem der Ansprüche 5 bis 8, wobei jedes dehnbare Element der Vielzahl von dehnbaren Elementen (62A, 62B, 62C) eine Vielzahl von Elektroden (17) einschließt, und wobei Elektroden der dehnbaren Elemente, die in dieselbe Richtung weisen, miteinander kurzgeschlossen sind.

10. Endovaskuläre medizinische Vorrichtung (50, 60) nach einem der Ansprüche 1 bis 9, wobei das dehnbare Element (52, 62) ein Formgedächtnismaterial einschließt.

11. Endovaskuläre medizinische Vorrichtung (50, 60) nach einem der Ansprüche 1 bis 10, wobei das dehnbare Element (52, 62) ein elektrisch leitfähiges Material radial einwärts von einem elektrisch isolierenden Material einschließt, wobei mindestens ein Abschnitt des elektrisch isolierenden Materials entfernt wird, um das elektrisch leitfähige Material freizulegen.

12. Endovaskuläre medizinische Vorrichtung (50, 60) nach Anspruch 11, wobei das freigelegte elektrisch leitfähige Material mindestens eine Elektrode (17) der einen oder mehreren Elektroden definiert.

13. Endovaskuläre medizinische Vorrichtung (50, 60) nach einem der Ansprüche 11 oder 12, ferner umfassend ein Elektrodenmaterial, das mit dem dehnbaren Element (52, 62) verbunden ist und in elektrischem Kontakt mit dem freigelegten elektrisch leitfähigen Material steht.

14. Endovaskuläre medizinische Vorrichtung (50, 60) nach Anspruch 13, wobei das Elektrodenmaterial eines oder mehrere aus einer Spule oder einem Band einschließt.

## Revendications

1. Dispositif médical endovasculaire (50) comprenant :
un corps allongé (51) conçu pour être introduit dans un vaisseau sanguin crânien d'un patient ; et
un élément expansible (52) sur le corps allongé,
dans lequel l'élément expansible comporte une pluralité de boucles (54A, 54B, 54C),
dans lequel chaque boucle comporte une ou plusieurs électrodes (17A, 17B, 17C), et
dans lequel l'élément expansible est conçu pour s'étendre radialement vers l'extérieur d'une configuration d'administration à profil relativement bas à une configuration déployée afin de positionner la ou les électrodes pour administrer une stimulation électrique au tissu d'un cerveau d'un patient ou détecter un paramètre du patient à partir d'un emplacement à l'intérieur du vaisseau sanguin crânien, **caractérisé en ce que** chaque boucle définit une forme de pétale de fleur, et dans lequel la pluralité de boucles est répartie autour d'un périmètre externe du corps allongé.

2. Dispositif médical endovasculaire (50) selon la revendication 1, dans lequel au moins deux électrodes (17A, 17B) d'au moins une boucle (54A) de la pluralité de boucles (54A, 54B, 54C) sont activables indépendamment.

3. Dispositif médical endovasculaire (50) selon l'une quelconque des revendications 1 ou 2, dans lequel au moins deux électrodes (17A, 17B) d'au moins une boucle (54A) de la pluralité de boucles (54A, 54B, 54C) sont en court-circuit avec l'une contre l'autre.

4. Dispositif médical endovasculaire (50) selon l'une quelconque des revendications 1 à 3, dans lequel chaque boucle de la pluralité de boucles (54A, 54B, 54C) comporte une pluralité d'électrodes (17A, 17B, 17C), et dans lequel les électrodes des boucles qui sont orientées dans une même direction sont court-circuitées ensemble.

5. Dispositif médical endovasculaire (60) selon l'une quelconque des revendications 1 à 4, dans lequel l'élément expansible (62) comporte une pluralité d'éléments expansibles (62A, 62B, 62C), chaque élément expansible comportant une ou plusieurs électrodes (17).

6. Dispositif médical endovasculaire (60) selon la revendication 5, dans lequel chaque élément expansible (62A, 62B, 62C) de la pluralité d'éléments expansibles (62) est séparé longitudinalement des éléments expansibles adjacents le long d'un axe longitudinal du corps allongé (64).

7. Dispositif médical endovasculaire (60) selon l'une quelconque des revendications 5 ou 6, dans lequel au moins deux électrodes (17) d'au moins un élément expansible (62A, 62B, 62C) de la pluralité d'éléments expansibles (62) sont activables indépendamment.

8. Dispositif médical endovasculaire (60) selon l'une quelconque des revendications 5 à 7, dans lequel au moins deux électrodes (17) d'au moins un élément expansible de la pluralité d'éléments expansibles (62A, 62B, 62C) sont court-circuitées l'une contre l'autre.

9. Dispositif médical endovasculaire (60) selon l'une quelconque des revendications 5 à 8, dans lequel chaque élément expansible de la pluralité d'éléments expansibles (62A, 62B, 62C) comporte une pluralité d'électrodes (17), et dans lequel des électrodes des éléments expansibles qui sont orientées dans une même direction sont couplées ensemble.

10. Dispositif médical endovasculaire (50, 60) selon l'une quelconque des revendications 1 à 9, dans lequel l'élément expansible (52, 62) comporte un matériau à mémoire de forme.

11. Dispositif médical endovasculaire (50, 60) selon l'une quelconque des revendications 1 à 10, dans lequel l'élément expansible (52, 62) comporte un matériau électriquement conducteur radialement vers l'intérieur d'un matériau électriquement isolant, dans lequel l'au moins une partie du matériau électriquement isolant est retirée pour exposer le matériau électroconducteur.

12. Dispositif médical endovasculaire (50, 60) selon la revendication 11, dans lequel le matériau électroconducteur exposé définit au moins une électrode (17) parmi la ou les électrodes.

13. Dispositif médical endovasculaire (50, 60) selon l'une quelconque des revendications 11 ou 12, comprenant en outre un matériau d'électrode connecté à l'élément expansible (52, 62) et en contact électrique avec le matériau conducteur d'électricité exposé.

14. Dispositif médical endovasculaire (50, 60) selon la revendication 13, dans lequel le matériau d'électrode comporte une ou plusieurs parmi une bobine ou une bande.
